# EUROPEAN PATENT SPECIFICATION

(11) **EP 2 760 995 B1**
(45) Date of publication and mention of the grant of the patent: **07.08.2019**
(21) Application number: 12835515.3
(22) Date of filing: 01.10.2012
(51) Int. Cl.: C12N 5/074, C12N 5/02, A61K 35/12, A61K 35/22

(54) **RENAL STEM CELLS ISOLATED FROM KIDNEY**
AUS DER NIERE ISOLIERTE NIERENSTAMMZELLEN
CELLULES SOUCHES RÉNALES ISOLÉES DU REIN

(30) Priority: 30.09.2011 US 201161541680 P
(43) Date of publication of application: 06.08.2014
(73) Proprietor: THE UNIVERSITY OF MIAMI, Miami, FL 33136 (US)
(72) Inventor: HARE, Joshua M., Miami Beach, FL 33141 (US); RANGEL, Erika, B, CEP 24026-001 Sao Paulo (BR); GOMES, Samirah, A, CEP 24026-001 Sao Paulo (BR)
(74) Representative: Predazzi, Valentina
(86) International application number: PCT/US2012/058369
(87) International publication number: WO 2013/049856

(56) References cited:
- WO-A1-2010/052192
- US-A1- 2011 091 428
- US-A1- 2011 091 428
- SCOTT REULE ET AL: "Kidney regeneration and resident stem cells", ORGANOGENESIS, vol. 7, no. 2, 1 April 2011 (2011-04-01), pages 135-139, XP055163934, ISSN: 1547-6278, DOI: 10.4161/org.7.2.16285
- SPANGRUDE G J ET AL: "A SIMPLIFIED METHOD FOR ENRICHMENT OF MOUSE HEMATOPOIETIC STEM CELLS", EXPERIMENTAL HEMATOLOGY, ELSEVIER INC, US, vol. 18, no. 8, 1 January 1990 (1990-01-01), pages 920-926, XP009031418, ISSN: 0301-472X
- BD Pharmingen: "BD Pharmingen(TM) Technical Data Sheet PerCP-Cy(TM)5.5 Mouse Lineage Antibody Cocktail, with Isotype Control Product Information", , XP055164480, Retrieved from the Internet: URL:http://www.bdbiosciences.com/ds/pm/tds /561317.pdf [retrieved on 2015-01-23]
- LAI L ET AL: "Mouse cell surface antigens: nomenclature and immunophenotyping", THE JOURNAL OF IMMUNOLOGY, THE AMERICAN ASSOCIATION OF IMMUNOLOGISTS, US, vol. 160, no. 8, 15 April 1998 (1998-04-15), pages 3861-3868, XP009145906, ISSN: 0022-1767
- ALEXANDRE, CHRISTIANNE SILVA ET AL.: 'Lineage-negative bone marrow cells protect against chronic renal failure' STEM CELLS vol. 27, no. 3, 02 March 2009, XP055082334
- LIU, DONGPING ET AL.: 'Puma is required for p53-induced depletion of adult stem cells' NATURE CELL BIOLOGY vol. 12, no. 10, October 2010, pages 1 - 14, XP055082335
- METSUYANIM, SALLY ET AL.: 'Expression of stem cell markers in the human fetal kidney' PLOS ONE vol. 4, no. 8, 2009, pages 1 - 15, XP002581532

## Description

### CROSS-REFERENCE TO RELATED APPLICATIONS

This application claims priority benefit of U.S. Application No. 61/541,680, filed September 30, 2011.

### STATEMENT REGARDING FEDERALLY SPONSORED RESEARCH

This invention was made with government support under NIH grant RO1 AG025017 awarded by the U.S. Department of Health and Human Services. The government has certain rights in the invention.

### FIELD OF THE INVENTION

The present invention relates to kidney-derived stem cells useful for renal development. Isolated stem cells may be used for in vivo replacement, regeneration, or repair of the kidney in a patient.

### BACKGROUND OF THE INVENTION

Chronic kidney disease is increasing at a rate of 6-8% annually in the United States alone. At present, dialysis and transplantation remain the only treatment options. There is hope, however, that stem cell therapy may provide an additional therapeutic approach for kidney disease.

The search for putative stem cells within the kidney has been the focus of extensive research. The identification of a renal stem cell would provide important biological insights and could be brought to bear therapeutically to generate new tubular, glomerular, and vascular cells in treatment of acute or chronic kidney injuries.

Several possible stem cell candidates have been described in kidney. These include label retaining cells (LRCs) or slow-cycling cells identified by using bromodeoxyuridine pulse-chase analyses, which were detected in interstitium, proximal tubules, thick ascending limb of Henle, distal tubules, and collecting ducts (Maeshima et al., 2003; Oliver et al., 2004; Maeshima et al., 2006). Other candidates include kidney cells expressing surface markers and found in different anatomical locations: interstitium (Seal) (Hishikawa et al., 2005; Dekel et al., 2006), Bowman's capsule (CD24 and CD133) (Sagrinati et al., 2006; Lazzeri et al., 2007; Ronconi et al., 2009; Appel et al., 2009), papilla (nestin and CD133) (Ward et al., 2011), and proximal tubular compartment (CD24 and CD133) (Sallustio et al., 2010; Lindgren et al., 2011) (or only CD133) (Bussolati et al., 2005). These studies demonstrated multipotentiality in vitro, and the capacity of these cells to integrate into the kidney during development or in response to injury.

But kidney epithelial tubular regeneration has been the subject of intense debate between multiple hypotheses. Cell-tracking studies using transgenic mice provide strong evidence in favor of an intra-tubular regeneration source, suggesting that differentiated epithelial cells that survive acute injury undergo proliferative expansion (Lin et al., 2005; Humphreys et al., 2008). More recently, a study involving two-step sequences of nucleotide analogue pulses following murine ischemia-reperfusion injury further suggests an absence of kidney stem cell in the adult kidney (Humphreys et al., 2011). Furthermore, telomerase activity-expressing cells were reported in 5% of the LRCs, but are not involved in kidney repair (Song et al., 2011b).

These studies generated controversy in the field, because they challenged the significance of work from many groups investigating the existence and the role of putative post natal kidney stem cells. Studies by Lin et al. (2005) and Humphreys et al. (2008; 2011) did not provide conclusive evidence for the absence of post-natal kidney stem cell and they did not eliminate the possibility of a tubular stem cell population, possibly of limited potency. Those cells derived from the Six2⁺ cap mesenchyma or expressing kidney specific-cadherin would be identically labeled in the regenerating tubules. There is also evidence that in addition to LRCs, other cells in the renal papilla can proliferate and migrate (Oliver et al., 2009). Additionally, the SDF-1/CXCR4 axis is involved in papillary LRC activation after acute kidney injury (Oliver et al., 2012).

Studies of other organs have engendered similar controversy. In the pancreas, the major source of new β-cells during adult life and after pancreatectomy was proliferation of terminally differentiated β-cells rather than from multipotent stem cells (Dor et al., 2004). More recently, however, rare pancreas-derived multipotent precursor cells that form spheres, express insulin, and generate multiple pancreatic and neural cell types in vivo were observed in embryonic and adult mice (Smukler et al., 2011). The presence of differentiation markers was also described in human neuronal stem cells displaying morphologic and molecular characteristics of differentiated astrocytes (Alvarez-Buylla et al., 2002).

Expression of c-Kit, a receptor tyrosine kinase, is detected in differentiated cells that do not exhibit stem cell properties, such as mast cells, germ cells, melanocytes, gastrointestinal Cajal cells, fetal endothelial cells, and epithelial cells, including breast ductal, sweat gland, some cells of skin adnexa, and cerebellum neurons (Miettinen and Lasota, 2005). c-Kit has also been described as a marker of stem cells in many organs and tissues, such as bone marrow (Ogawa et al., 1993), amniotic fluid (De et al., 2007), lungs (Kajstura et al., 2011), heart (Beltrami et al., 2003), and liver (Crosby et al., 2001).

Improved processing and products produced thereby for kidney replacement, regeneration, or repair preparation are now described. Renal stem cells are characterized by expressing c-Kit⁺, maintaining stemness over several population doublings over time during in vitro culture, and differentiating into many different renal cell lineages.

Embryonic or induced pluripotent stem cells are not needed for the replacement of kidney function in a patient in need of treatment. Other advantages of the invention are discussed below or would be apparent to a person skilled in the art from that discussion.

### SUMMARY OF THE INVENTION

The present invention is defined in the claims.

It is an object of the invention to provide a novel population of stem cells, which are multipotent precursors of differentiated cells in the renal lineage, derived from kidney as defined in the claims. Renal stem cells express the receptor tyrosine kinase c-Kit. They are isolated from kidney (e.g., adult, neonatal, or fetal); kidney harvested from a living donor or cadaver is preferred. Cells are selected for expression of c-Kit (i.e., c-Kit⁺). Cells may be counter selected for non-expression of lineage markers (i.e. Lin⁻). Renal stem cells may be expanded by in vitro culture over several population doublings. They may be differentiated into many different endothelial or epithelial renal cell lineages.

In one embodiment, c-Kit⁺/Lin⁻ cells wherein the renal stem cells express Oct4 isolated from kidney tissue are provided as renal stem cells.

In another embodiment, the renal stem cells are for medical use according to the claims and can be administered, at least once, to a patient needing replacement of kidney function.

In yet another embodiment, the cells are for use in a method of kidney regeneration or repair comprising administering, at least once, to a patient in need of such treatment, a therapeutically effective amount of the renal stem cells. They can be expanded in vitro prior to administration.

Further aspects and advantages of the invention will be apparent to a person skilled in the art from the following detailed description and claims, and generalizations thereto.

### BRIEF DESCRIPTION OF THE DRAWINGS

Figure 1 is a schematic illustration of a process for c-Kit⁺ renal stem cells isolated from kidney then expanded in culture.
Figure 2 shows characterization of c-Kit⁺ renal stem cells. Cells were stained with specific-antibody and analyzed by FACS: negative control (Fig. 2A), c-Kit vs. Oct4 (Fig. 2B), c-Kit vs. Sox2 (Fig. 2C), c-Kit vs. Klf4 (Fig. 2D), c-Kit vs. c-Myc (Fig. 2E), and c-Kit vs. Six2 (Fig. 2F).
Figure 3 shows gene expression detected by quantitative real-time PCR and analyzed by the fold change (2^{ΔΔCt}) in undifferentiated c-Kit⁺/Lin⁻ renal stem cells over neonatal rat kidney. Bar represents mean ± SEM.

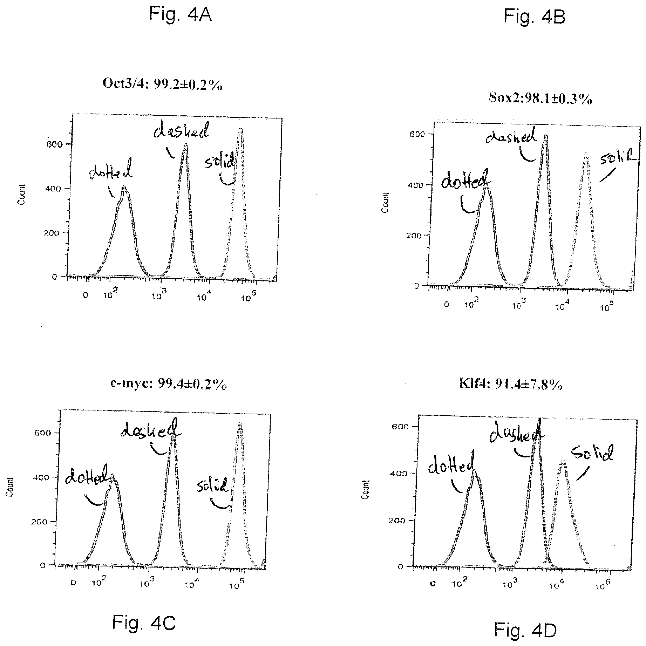

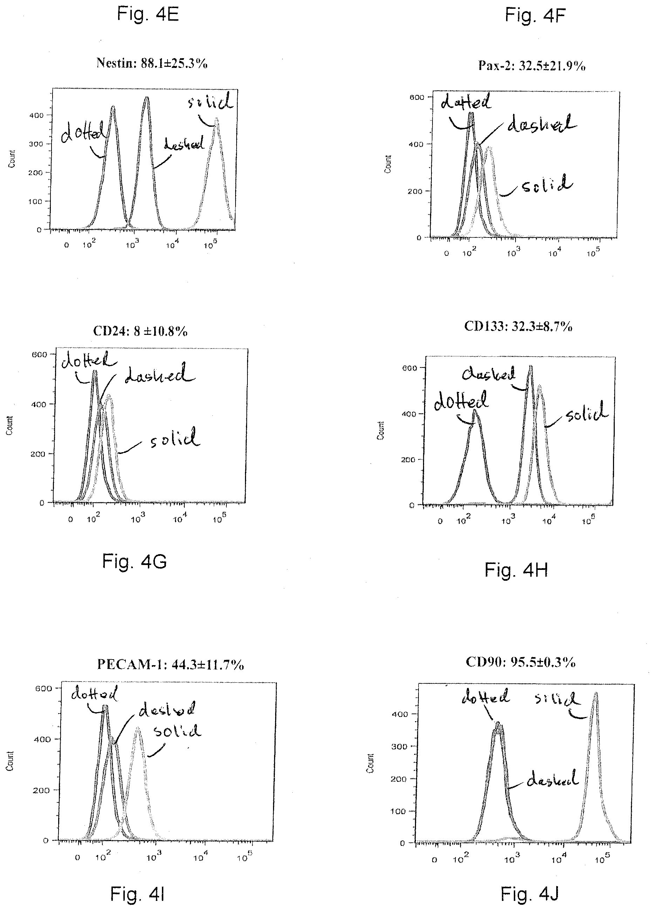

Figure 4 shows flow cytometric analysis of c-Kit⁺/Lin⁻ renal stem cells. Horizontal line on each histogram indicates the proportion of positive cells (solid line) for each intra-nuclear, intracellular, or surface protein as compared to unstained cells (dotted line) and to secondary antibody alone (dashed line) negative controls. All secondary antibodies were Alexa-Fluor 568 except for CD90-conjugated FITC. Value is the percentage of positive cells detected as mean ± SD for: octamer-binding POU transcription factor 4 (Oct4 in Fig. 4A), sex-determining-region Y-box 2 (Sox2 in Fig. 4B), basic helix-loop-helix leucine-zipper transcription factor (c-Myc in Fig. 4C), Kruppel-like factor 4 (Klf4 in Fig. 4D), a type IV intermediate filament (nestin in Fig. 4E), transcription factor paired box 2 (Pax2 in Fig. 4F), adhesion molecule heat stable antigen (CD24 in Fig. 4G), prominin 1 (CD133 in Fig. 4H), platelet-endothelial cell adhesion molecule (PECAM-1 in Fig. 4I), and Thy-1 (CD90 in Fig. 4J).

### DESCRIPTION OF SPECIFIC EMBODIMENTS OF THE INVENTION

The presence of tissue specific precursor cells is an emerging concept in organ formation and tissue homeostasis. Several candidates were described in the kidney, but their identification as true stem cells remained elusive. We hypothesized that c-Kit⁺ cells isolated from kidney could be renal stem cells. Here, we demonstrate that c-Kit⁺ cells possess renal stem cell properties, including self-renewal capacity, clonogenicity, and multipotentiality. They have the potential to treat renal failure by multicompartment engraftment, e.g., tubular, vascular, and glomerular, to promote endogenous repair in acute ischemia-reperfusion injury, and otherwise be useful in treatment of kidney disease (especially glomerular diseases and acute tubular necrosis).

The kidney-derived c-Kit⁺ cell population isolated below fulfills all of the criteria for a renal stem cell. These cells originate in the thick ascending limb of Henle's loop and exhibit clonogenicity, self-renewal, and multipotentiality with differentiation capacity into mesodermic and ectodermic lineages. The c-Kit⁺ cells formed spheres in non-adherent conditions when plated at clonal density and expressed markers of stem, precursor, or differentiated cells. Ex vivo expanded c-Kit⁺ cells integrated into several compartments of the kidney, including tubules, vessels, and glomeruli, and contributed to functional and morphological improvement of the kidney following acute ischemia-reperfusion injury in rats. Together these findings documenting a novel kidney-derived c-Kit⁺ cell population that can be isolated, expanded, cloned, differentiated, and employed for kidney repair following acute kidney injury have important biological and therapeutic implications.

Embodiments of the invention are directed to isolated renal stem cells and purified stem cell compositions, which are c-Kit⁺/Lin⁻ cells and express Oct4 as defined in the claims. These stem cells are multipotent precursors present in kidney that can be isolated, expanded, and committed to a differentiated renal cell type. Moreover, they are suitable for tissue replacement due to their organ-specific identity, which obviates the need for directed differentiation. Other embodiments are directed to inducing differentiation of renal stem cells towards epithelial or endothelial cells, providing efficacious treatment of kidney diseases or disorders. In particular, the risk is reduced for tumorigenic potential (i.e., transformed cells) or pathologies that are of concern for other cell types.

The invention is described with reference to the drawings that are attached. Several aspects of the invention are described below with reference to example applications for illustration. It should be understood that numerous specific details, relationships, and methodologies are set forth to provide a full understanding of the invention. One having ordinary skill in the relevant art, however, would readily recognize that the invention can be practiced without one or more of the specific details or with other methods. The present invention is not limited by the illustrated ordering of acts or events, as some acts may occur in different orders and/or concurrently with other acts or events. Furthermore, not all illustrated acts or events are required to implement a methodology in accordance with the present invention.

Embodiments of the invention may be practiced without the theoretical aspects presented. Moreover, the theoretical aspects are presented with the understanding that they do not limit the invention unless they are explicitly recited in the claims.

Unless otherwise defined, all terms herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

### Definitions

The terminology used herein is for the purpose of describing particular embodiments only and is not intended to be limiting of the invention. As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising."

The term "about" or "approximately" means within an acceptable error range for the particular value as determined by the skilled artisan, which will depend in part on how the value is measured (i.e., limitations of the measurement system). For example, "about" can mean within one standard deviation per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value. Where particular values are described in the specification and claims, unless otherwise stated the term "about" should be assumed to mean within an acceptable error range for that measurement.

As used herein, the term "autologous" is meant to refer to any material derived from the same patient to whom it is later to be reintroduced into the patient. The term "allogeneic" refers to the same human species but genetically different in one or more genetic loci from the patient. The term "xenogeneic" refers to an animal species other than the human species of the patient.

The term "patient" refers to a human subject. In some cases, the methods of the invention find use in experimental animals, in veterinary application, and in the development of animal models for disease, including, but not limited to, pigs, primates, and rodents (e.g., mice, rats, and hamsters).

"Diagnostic" or "diagnosed" means identifying the presence or nature of a pathologic condition. Diagnostic methods differ in their sensitivity and specificity. The "sensitivity" of a diagnostic assay is the percentage of affected subjects who test positive (i.e., "true positives"), whereas affected subjects not detected by the assay are "false negatives." Subjects who are not affected and who test negative in the assay are "true negatives." The "specificity" of a diagnostic assay is 1 minus the false positive rate, where the false positive rate is defined as the percentage of unaffected subjects who test positive. While a particular diagnostic method may not provide a definitive diagnosis of a condition, it suffices if the method provides a positive indication that merely aids in diagnosis.

"Treatment" is an intervention performed with the intention of preventing the development or altering the pathology or symptoms of a kidney disease or disorder. Accordingly, "treatment" refers to both therapeutic treatment and preventative measures to treat a kidney disease or disorder. Those in need of treatment include those already with the kidney disease or disorder as well as those in which the kidney disease or disorder is to be prevented. As used herein, "ameliorated" or "treatment" refers to a kidney disease or disorder's symptom that approaches a normalized value (for example a value obtained in a healthy patient or individual), e.g., is less than 50% different from a normalized value, preferably is less than about 25% different from a normalized value, more preferably, is less than 10% different from a normalized value, and still more preferably, is not significantly different from a normalized value as determined using routine statistical tests.

A "therapeutically effective amount" or "effective amount" of renal stem cells or secretion products of cultured renal stem cells, as the terms are used herein, is an amount sufficient to treat a patient. The effective amount for any particular patient will be determined by a physician taking into account such factors as patient general health and age, severity of the condition being treated, body weight, and the like.

### Renal Stem Cells

In a preferred embodiment, a cell culture comprises isolated c-Kit⁺/Lin⁻ renal stem cells. Preferably, the c-Kit⁺/Lin⁻ renal stem cells comprise precursor cells capable of differentiating into multiple cell lineages, including endothelial, epithelial, mesenchymal, and neuronal cells.

In another preferred embodiment, an isolated cell comprises a c-Kit⁺/Lin⁻ renal stem cell, which is a precursor cell capable of differentiating into multiple cell layers, including ectodermal, mesodermal, and endodermal tissues.

A method of producing a renal stem cell, which comprises isolating and purifying c-Kit⁺/Lin⁻ cells from kidney tissue; and culturing and expanding the c-Kit⁺/Lin⁻ cells in vitro. Preferably, the renal stem cells comprise multipotent precursor cells.

In one embodiment, the hematopoietic lineage of stem cells is removed from the culture before, during, and/or after isolation of the c-Kit⁺ stem cells. Hematopoietic lineage cells identified by one or more of markers CD3e, CD11b, CD45R/B220, Ly-76, Ly-6G, Ly-6C, and CD117 (especially their human homologs) may be removed at some point(s) during production. Expression of the markers CD45, nephrin, poducin, E-cadherin, podocin, CD326, angiotensin receptor type Ia, angiotensin receptor type II, 0and mast cell tryptase was not detected by quantitative real-time PCR.

The c-Kit⁺/Lin⁻ renal stem cells express over time at least one marker comprising: octamer-binding POU transcription factor Oct4, sex-determining-region Y-box 2 (Sox2), basic helix-loop-helix leucine-zipper transcription factor c-Myc, Kruppel-like factor 4 (Klf4), or Musashi. Preferred is the expression of at least any two, at least any three, or at least all four of markers Oct4, Sox2, c-Myc, and Klf3.

The c-Kit⁺/Lin⁻ renal stem cells are multipotent and can differentiate into multiple different endothelial or epithelial lineages.

The c-Kit⁺/Lin⁻ derived cell lineage comprises at least one endothelial cell marker comprising: CD31, CD34, von Willebrand factor (vWF), vascular endothelial growth factor (VEGFa), or smooth muscle actin (SMA).

The c-Kit⁺/Lin⁻ derived cell lineage comprises at least one epithetlial marker comprising: wingless-type 4 (Wnt4), Na-K-2CI co-transporter (NKCC2), Na-Cl co-transporter (NCCT), Wilm's tumor suppressor gene-1 (WT-1), cytokeratin 18 (KRT18), zona occludens-1 (ZO-1), β-catenin, neurogenic Notch homolog 2 (Notch2), aquaporin 1 (AQP1), or cadherin 6.

The c-Kit+/Lin- derived cell lineage comprises at least one mesenchymal marker comprising: CD73, CD90, CD105, CD146, vimentin, CD24, CD133, or sine oculis-related homeobox 2 (Six2).

The c-Kit⁺/Lin⁻ derived cell lineage comprises at least one neuronal marker comprising: CD56, β-3 tubulin, synaptopodin, nestin, or neurofilament.

In another preferred embodiment, a method of treating kidney diseases or disorders comprising administering to a patient in need thereof, a therapeutically effective amount of c-Kit⁺/Lin⁻ stem cells and/or c-Kit⁺/Lin⁻ stem cell secretion products.

In another preferred embodiment, a method of repairing and regenerating kidney tissue in vivo, comprising administering to a patient in need thereof, a therapeutically effective amount of c-Kit⁺/Lin⁻ stem cells and/or c-Kit⁺/Lin⁻ stem cell secretion products.

The renal stem cell may be autologous, allogeneic, or xenogeneic in relation between the patient recipient and the kidney donor.

Isolated renal stem cells, purified renal stem cell compositions, renal stem-cell secreted products, or any combination thereof, may be used in the repair of a patient's kidney, damaged by any cause including, for example, disease, injury, genetic abnormalities, shock, obesity, wounds (physical or chemical), cirrhosis, and the like.

An early stage of any renal impairment can be treated irrespective of the specific pathology of the kidney dysfunction prevailing. For example, the early stage of renal impairment may be an early stage glomerulonephritis, an early stage polycystic kidney disease, an early stage chronic pyelonephritis, or an early stage diabetic nephropathy.

Other examples of kidney disease or disorder comprise: acute tubular necrosis, chronic renal failure, renal hypertrophy, renal hyperplasia, terminal kidney disease, glomerulonephritis, or the like.

Renal stem cells or secreted products thereof can be used alone as single therapy or in combination with another therapy. For example, administration of angiotensin converting enzyme inhibitors as an adjunct to prevent, reduce, or reverse loss of renal function.

Patients in need of treatment can be diagnosed by any means known to those of ordinary skill in the art. For example, chronic renal failure (CRF) may result from any major cause of renal dysfunction. The functional effects of CRF can be categorized as diminished renal reserve, renal insufficiency (failure), and uremia. Plasma concentrations of creatinine and urea begin a nonlinear rise as renal function diminishes. Sodium ion (Na⁺) and water balance is well maintained by increased fractional excretion of Na⁺ and a normal response to thirst. Thus, the plasma Na⁺ concentration is typically normal and hypervolemia is infrequent despite unmodified dietary intake of Na⁺. But imbalances may occur if Na⁺ and water intakes are restricted or excessive.

Causes of CRF include, without limitation, glomerulopathies, e.g., IgA nephropathy, focal glomerulosclerosis, membranous nephropathy, membranoproliferative glomerulonephritis, idiopathic crescentic glomerulonephritis, diabetes mellitus, postinfectious glomerulonephritis, systemic lupus erythematosus, Wegener's granulomatosis, hemolytic-uremic syndrome, amyloidosis; chronic tubulointerstitial nephropathies; hereditary nephropathies, e.g., polycistic kidney disease, Alport's syndrome, medullary cystic disease, Nail-patella syndrome; hypertension, e.g., nephroangiosclerosis, malignant glomerulosclerosis; renal macrovascular disease; and obstructive uropathy, e.g., ureteral obstruction, vesicoureteral reflux, benign prostatic hyperplasia; and the like.

Patients with mildly diminished renal reserve are asymptomatic, and renal dysfunction might only be detected by laboratory testing. A patient with mild to moderate renal insufficiency may have only vague symptoms despite elevated BUN and creatinine; nocturia is noted, principally due to a failure to concentrate the urine during the night. Lassitude, fatigue, and decreased mental acuity often are the first manifestations of uremia.

Stem Cell Biology: There are many undifferentiated cells found in vivo. Stem cells are undifferentiated immature cells, capable of self renewal (division without limit) and differentiation (specialization). These juvenile cells are abundant in a developing embryo; however, their numbers decrease as development progresses. By contrast, an adult organism contains limited number of stem cells which are confined to certain body compartments.

Stem cells may be monopotent, bipotent, multipotent, or totipotent. Monopotent and bipotent precursor cells are more restricted in development and give rise to one or two types of specialized cells, respectively. In contrast, multipotent precursor cells can differentiate into many different types of cells, giving rise to tissue (which constitute organs) or in the case of totipotent precursor cells, the whole organism. Multipotent precursor cells, unlike monopotent or bipotent, are capable of multilineage differentiation, giving rise to a tissue that would consist of a collection of cells of different types, layers, or lineages.

According to the current understanding, a stem cell, such as a multipotent precursor cell, has the following four characteristics: (i) it is an undifferentiated cell (i.e., not terminally differentiated), (ii) it has the ability to divide without limit, (iii) it has the ability to give rise to differentiated progeny, and (iv) when it divides each daughter has the choice of either maintaining stemness like its parent or committing to a differentiated type of cell.

The hematopoietic stem cell is an example of a multipotent stem cell which is found among marrow cells and gives rise to all the various blood cells (including leucocytes and erythrocytes). Hematopoietic stem cells can be extracted by isolation from bone marrow, growth factor mobilized peripheral blood, or cord blood. Recently, hematopoietic stem cells have been prepared from embryonic stem (ES) cells, which are extracted from embryos obtained using in vitro fertilization techniques. These undifferentiated cells are capable of multilineage differentiation and reconstitution of all body tissue (i.e., totipotent).

ES cells are characterized by several known markers such as stage-specific embryonic markers 3 and 4 (SSEA-3 and SSEA-4), high molecular weight glycoproteins TRA-1-60 and TRA-1-81, and alkaline phosphatase. These markers can be used to distinguish renal stem cells from ES or induced pluripotent stem (iPS) cells.

Cellular Antigens: Various antigens are associated with either undifferentiated and differentiated cells. The term "associated" here means the cells expressing or capable of expressing, or presenting or capable of being induced to present, or comprising, the respective antigen(s). Each specific antigen associated with an undifferentiated cell or a differentiated cell can act as a marker. Hence, different types of cells can be distinguished from each other on the basis of their associated particular antigen(s) or on the basis of a particular combination of associated antigens.

Some of the markers identified on myeloid stem cells comprise CD34⁺, DR⁺, CD13⁺, CD33⁺, CD7⁺, and TdT⁺ cells. PSCs are CD34⁺, DR⁻, and TdT⁻ cells (other useful markers being CD38⁻ and CD36⁺). LSCs are DR⁺, CD34⁺, and TdT⁺ cells (also CD38⁺). ES cells express SSEA-3 and SSEA-4, high molecular weight glycoproteins TRA-1-60 and TRA-1-81 and alkaline phosphatase. They also do not express SSEA-1, the presence of which is an indicator of differentiation. Other markers are known for other types of stem cells, such as nestin for neuroepithelial stem cells. Mesenchymal stem cells are also positive for SH2, SH3, CD29, CD44, CD71, CD90, CD106, CD120a, and CD124, for example, and negative for CD34, CD45, and CD14.

Alternatively, or in addition, many cells can be identified by morphological characteristics. The identification of cells using microscopy, optionally with staining techniques is an extremely well developed branch of science termed histology and the relevant skills are widely possessed in the art.

Various techniques may be employed to separate the cells by initially removing cells of dedicated lineage. Monoclonal antibodies are particularly useful for identifying markers associated with particular cell lineages and/or stages of differentiation.

If desired, a large proportion of non-desired cells (e.g., terminally differentiated) may be removed by initially using a "relatively crude" separation. For example, isolation using culture of kidney explant cells and immunoselection (e.g., immunopanning, magnetic bead separation, FACS, or any combination thereof) may be initially used to remove large numbers of lineage committed cells. Desirably, at least about 50%, at least about 60%, at least about 70%, at least about 80%, at least about 90%, or at least about 95%, or at least about 99% of all cells in the kidney explant culture are removed during enrichment of renal stem cells.

Procedures for immunoselection include, but are not limited to, panning with antibody attached to a solid matrix (e.g., culture plate), separation using antibody-coated magnetic beads, affinity chromatography, cytotoxic agent joined to a monoclonal antibody, elutriation, or any combination thereof. Another technique is automated flow cytometry, which can have varying degrees of sophistication, e.g., a plurality of color channels, low angle and obtuse light scattering detecting channels, impedance channels, etc.

Tissue-specific markers can be detected using any suitable immunological technique: such as flow immunocytochemistry or affinity adsorption for cell-surface markers, immunocytochemistry (for example, of fixed cells or tissue sections) for intracellular or cell-surface markers, Western blot analysis of cellular extracts, and enzyme-linked immunoassay, for cellular extracts or soluble products secreted into the medium. Expression of an antigen by a cell is said to be antibody-detectable if a significantly detectable amount of antibody will bind to the antigen in a standard immunocytochemistry or flow cytometry assay, optionally after fixation and/or permeablization of the cells, and possibly using a labeled secondary antibody or other conjugate (such as a biotin-avidin conjugate) to amplify labeling.

Expression of tissue-specific gene products can be detected at the RNA level by Northern blot analysis, dot-blot hybridization analysis, or by real time polymerase chain reaction (RT-PCR) using sequence-specific primers in standard amplification methods. See US 5,843,780 for details of general techniques. Sequence data for other markers listed in this disclosure can be obtained from public databases such as GenBank. Expression at the RNA level is said to be detectable according to one of the assays described herein if a clearly discernable hybridization or amplification product results. Expression of tissue-specific markers detected at the protein or RNA level is considered positive if the level is at least 2-fold, and preferably more than 10- or 50-fold above that of a negative control cell.

Once markers have been identified on the surface of cells of the desired phenotype, they can be used for immunoselection to further enrich the population by techniques such as immunopanning or antibody-medicated fluorescence-activated cell sorting.

### Treatment of Patient

The amount of renal stem cells administered to a patient will vary depending on the patient's condition and disease, and would be determined by consideration of all appropriate factors by the medical practitioner. Preferably, however, about 1x10⁶ to about 1x10¹², about 1x10⁸ to about 1x10¹¹, or about 1x10⁹ to about 1x10¹⁰ stem cells may be utilized for an adult human. This amount may vary depending on the patient's age, weight, size, condition, and gender; the specific kidney disease or disorder to be treated; route of administration, whether administration is localized to the kidney (e.g., depot) or systemic circulation (e.g., infusion); and other factors. Preferably, renal stem cells may be introduced intravenously or in a renal artery, and then localize to the patient's kidney. Those skilled in the art should be readily able to derive appropriate dosages and dosing schedules of administration to suit the particular circumstance and needs of the patient.

Methods of re-introducing cellular components are known in the art (see US 4,844,893 and US 4,690,915) and they may be used to administer renal stem cells to a patient.

### Pharmaceutical Compositions

In other embodiments, the present invention provides pharmaceutical compositions comprising renal stem cells as defined in the claims. As compared to the total number of cells in the composition, at least about 50%, at least about 60%, at least about 70%, at least about 80%, at least about 90%, or at least about 95%, or at least about 99% are renal stem cells. The composition may be comprised of at least about 10⁶, at least about 10⁷, at least about 10⁸, at least about 10⁹, at least about 10¹⁰, or at least about 10¹¹ renal stem cells. The concentration of renal stem cells in the composition may be at least about 10⁷/ml, at least about 10⁸ml, or at least about 10⁹/ml. In other preferred embodiments, pharmaceutical compositions comprise the renal stem cells' secreted products.

In other aspects, kits are provided for ameliorating renal tissue damage or for delivering a functional gene or gene product to the kidney of a patient comprising renal stem cells. The cells may be transfected or transduced with nucleic acid provided in the kit. Stem cells generally have been administered by injection into the patient's kidney or infusion into the patient's local or systemic circulation.

In some embodiments, administration of the stem cell compositions can be coupled with other therapies. For example, a therapeutic agent can be administered prior to, concomitantly with, or after administering renal stem cells to a patient.

Pharmaceutically acceptable carriers are determined in part by the particular composition being administered, as well as by the particular method used to administer the composition. Accordingly, there is a wide variety of suitable formulations of pharmaceutical compositions of the present invention. Renal stem cells may be formulated with a pharmaceutically accept-able carrier. Suitable methods of administering such cells to a patient are available (e.g., injection or infusion).

Formulations suitable for parenteral administration, such as, for example, by injection or infusion (e.g., intravenous, intraperitoneal, or subcutaneous routes), include aqueous and non-aqueous, isotonic sterile injection solutions, which can contain buffers, bacteriostats, and solutes that render the formulation isotonic with the body fluid of the patient. The formulation can be presented in unit-dose or multi-dose sealed containers, such as ampules and vials.

Extemporaneous injection solutions and suspensions can be prepared from sterile powders, granules, and tablets of the kind previously described. The dose administered to a patient should be sufficient to provide a beneficial therapeutic response in the patient over time. The dose will be determined by the efficacy of the renal stem cells employed and the condition of the patient, as well as the body weight of the patient to be treated. The dosage amount and dosing schedule will be determined by the existence, nature, and extent of any adverse side effects that accompany the administration of the renal stem cells in a particular patient.

Administration of renal stem cells transfected or transduced ex vivo can be by any route usually used for introducing cells into a patient. Transduced cells may be prepared for reinfusion according to established methods (see Abrahamsen et al., J. Clin. Apheresis 6, 48-53, 1991; Carter et al., J. Clin. Apheresis 4,113-117, 1988; Aebersold et al., J. Immunol. Methods 112, 1-7, 1988; Muul et al., J. Immunol. Methods 101, 171-181, 1987; and Carter et al., Transfusion 27, 362-365, 1987). After a period of about 2-4 weeks in culture, the cells may number between 1×10⁶ and 1×10¹⁰. In this regard, the growth characteristics of cells vary from patient to patient and from cell type to cell type. About 72h prior to reinfusion of the transduced cells, an aliquot is taken for analysis of phenotype and the percentage of cells expressing the heterologous gene product (e.g., green fluorescent protein) is determined. Renal stem cells transduced for ex vivo therapy can be administered parenterally as described above. In determining the effective amount of cells to be administered for treatment or prophylaxis, the medical practitioner should evaluate circulating plasma levels, and, in the case of replacement therapy, the production of the heterologous gene product.

Renal stem cells can be administered at a rate determined by the ED₅₀ for the biological or therapeutic effect and any side effect at various concentrations, as applied to the age, weight, size, condition, and gender of the patient. Administration can be accomplished via single or divided doses. Adult stem cells may also be mobilized using exogenously administered factors that stimulate their production and egress from kidney.

The following examples are meant to be illustrative of the present invention, but the practice of the invention is not limited or restricted in any way by them.

### EXAM PLES

### Materials and Methods

Explant Culture of Neonatal Rat Kidney: Neonatal rat kidneys were harvested, minced, digested with collagenase II, and incubated in red blood cell lysing buffer (Sigma-Aldrich). After washing, the explanted cells were plated and expanded in DMEM/F12 medium supplemented with 20% fetal bovine serum (FBS), 100U/ml penicillin, and 100µl/ml streptomycin (Sigma-Aldrich) for two weeks. This medium was changed every other day. All cells were cultured at 37°C in 98% humidified air containing 5% CO₂.

Isolation of c-Kit⁺/Lin⁻ Cells: Kidney-derived cells were isolated from the explant culture by immunopanning using rabbit polyclonal c-Kit antibody (H300, Santa Cruz) and further selected by fluorescence-activated cell sorting (BD FACSAria™, University of Miami). Depletion of hematopoietic stem cells was also performed to ensure that c-Kit⁺ cells come from the kidney and that bone marrow-derived cells were removed. For this purpose, APC lineage antibody cocktail was used, which depletes CD3e, CD11b, CD45R/B220, Ly-76, Ly-6G and Ly-6C (BD Pharmigem), and anti-mouse CD117-PE conjugated (eBioscience) to counter select for those lineages.

Expansion of c-Kit⁺/Lin⁻ Cells: Isolated c-Kit⁺/Lin⁻ cells were plated and expanded in DMEM/F12 medium supplemented with 10% FBS, 10ng/ml basic fibroblast growth factor (bFGF), 20ng/ml epidermal growth factor, 10ng/ml recombinant leukemia inhibitory factor (Millipore), 40ng/ml stem cell factor (PreproTech), insulin-transferrin-selenium (Invitrogen), and antibiotics.

Characterization of c-Kit⁺/Lin⁻ Cells: For real-time (RT) PCR, total RNA was extracted from cells using Pure-Link Micro-to-Midi total RNA purification system (Invitrogen) and reverse transcribed using High Capacity cDNA reverse transcription kit (Applied Biosystems). All samples were treated with TURBO DNase (Ambion). RT-PCR was performed in triplicate using a 20µl reaction mixture containing lOng cDNA, TaqMan Universal PCR Master Mix (Roche) and primers/probes sets for c-Kit, Six2, CD24, CD133, Oct4, Sox2, Klf4, c-MYC, Flk1, vWF, Acta2, VEGFa, PECAM-1, CD31, Wnt4, β-catenin, Notch2, nestin, β-3 tubulin, neurofilament heavy chain (NF-H), synaptopodin, CD56, CD73, CD90, CD105, CD146, E-cadherin, cadherin-6, cytokeratin 18, zona occludens-1, CD326, CD45, and CD34 (TaqMan gene expression assay, Applied Biosystems). As an internal control, glyceraldehyde 3-phosphate dehydrogenase (GAPDH) or 18S RNA was quantified in each reaction. Reaction conditions were performed according to the manufacturer: 50°C for 2m, 90°C for 10m, and 40 cycles at 95°C for 15s and 60°C for 1m. Software from iQ5 multicolor RT-PCR detection system (Bio-Rad) was used for PCR analyses. Relative fold change for quantitative real-time (q) PCR was calculated by 2^{ΔΔCt} method and compared to baseline values (set at 1).

For immunofluorescence, the cells were fixed in paraformaldehyde 4% for 15m at room temperature. Blocking solution (1% BSA and 0.5% Tween-20) was used for 1h at room temperature. The samples were then incubated overnight at 4°C with specific primary antibodies for mouse monoclonal anti-actin alpha smooth muscle (Sigma-Aldrich), rabbit polyclonal anti-beta tubulin 3 chain, mouse monoclonal anti-CD31, rabbit polyclonal anti-vWF, rabbit polyclonal anti-N-cadherin (all from Abcam), and monoclonal anti-mouse SCF R/c-Kit/CD1117 (R&D) diluted from 1:50 to 1:100. After washing in PBS, incubations were performed at room temperature for 1h using 488- or 568-conjugated secondary antibodies (Invitrogen) at a dilution from 1:200 to 1:250. Nuclei labeling was obtained with 4'-6-diamidino-2-phenylindole (DAPI) after incubation for 15m at room temperature. Slides were mounted in ProLong Gold antifade reagent (Invitrogen). Images were obtained using a Zeiss LSM-710 confocal microscope (Analytical Imaging Core Facility, University of Miami).

For FACS of intra-nuclear markers, cells were fixed in cold 80% ethanol, washed twice with 1ml PBS during centrifugation for 10m at 200g, incubated 1h with FACS buffer (1% BSA and 5% FBS diluted with distilled water) on ice, and subsequently 1h with primary and secondary antibodies (washed thrice for 5m during centrifugation between primary and secondary, and after secondary). BD Cytofix/ Cytoperm fixation/permeabilization kit (BD Pharmigem) was used for intra-cellular markers. For surface markers, periods of incubation with FACS buffer for 1h, and incubations with primary and secondary antibodies were also performed.

Clonogenicity and Self-Renewal: Clonogenicity was assessed in a 96-well plate by performing two serial dilutions. Briefly, 2×10⁴ cells in 200µl were added to well A1 and 100µl were quickly transferred to well B1 and mixed gently by pipetting. Using the same tip, the 1:2 dilution was repeated down the entire column, discarding 100µl from H1 so that it ends up with the same volume as the wells above. With the multi-channel pipettor, an additional 100µl of medium was added in column (giving a final volume of cells and medium of 200µl). Then using the same pipettor 100µl was quickly transferred from the wells in the first column (A1 through H1) to those in the second column (B2 through H2) and mixed by gently pipetting. The 1:2 dilutions were repeated across the entire plate. The final volume of all wells was brought to 200µl by adding 100µl of medium to each well. The plate was incubated at 37°C in 98% humidified air containing 5% CO₂. Single-cell deposition was confirmed microscopically and wells containing more than one cell were excluded. Clones were observed 4-5 days after cells were plated. After ∼ 2 weeks, colonies developed and were expanded. Three of those colonies were subcultured in larger vessels for at least 15 passages without evidence of senescence.

Self-renewal was analyzed by the measurement of telomerase activity in the early and late passages. For this purpose, we used the TRAPeze® XL telomerase detection kit (Millipore), which incorporates the use of the novel Amplifluor fluorescence energy transferlabeled primers, so that quantitative measurements are obtained.

In Vitro and In Vivo Multipotency of c-Kit⁺ Cells: Besides endothelial differentiation (mesoderm), neuronal (ectoderm) or hepatocyte (endoderm) differentiation will be performed by growing the c-Kit⁺ cells with 100ng/ml bFGF or 10ng/ml FGF-4 and 20ng/ml HGF, for 2 weeks, respectively. For osteogenic differentiation, c-Kit cells were cultured in α-MEM and 10% FBS that contained 10⁻⁷ M dexamethasone, 0.2mM ascorbic acid, and 10mM β-gtycero-phosphate (all from Sigma-Aldrich). The medium was changed twice a week for 3 weeks. For adipogenic differentiation, c-Kit⁺ cells were incubated in DMEM high glucose (Invitrogen,) that contained 10% FBS, 1µM dexamethasone, 0 .5µM 1-methyl-3-isobuthylxanthine, 10µg/mL insulin, and 100µM indomethacin (all from Sigma-Aldrich). After 72h, the medium was changed do DMEM high glucose, 10% FBS, and 10µg/ mL insulin for 24h. These treatments were repeated three times. c-Kit⁺ stem cells (5×10⁶ cells embedded in MATRIGEL extracellular matrix substrate), are injected into the thigh muscle, to assess if these cells can form teratomas, which represent the three germ layers (ectoderm, mesoderm, and endoderm), after 4 weeks in non-obese diabetic severe combined immunodeficiency (NOD-SCID) mice.

In Vitro Differentiation: Early (< P25) or late passages (> P40) of c-Kit⁺/Lin⁻ cells were analyzed. For endothelial differentiation, cells were plated and cultured in Endothelial Cell Basal Medium-2 (Lonza) supplemented with 2% FBS, EGF, VEGF, IGF, bFGF, hydrocortisone, ascorbic acid, and heparin for 1-4 weeks. For epithelial differentiation, cells were incubated in DMEM containing 10% FBS, 50ng/ml bFGF, 20ng/ml LIF, and 5ng/ml TGF-β3 for 3 weeks. For adipogenic differentiation, cells were incubated in DMEM high glucose containing 10% FBS, 1µM dexamethasone, 0.5µM 1-methyl-3-isobuthylxanthine, 10µg/mL insulin, and 100µM indomethacin (Sigma-Aldrich) for 2 weeks. For osteogenic differentiation, cells were cultured in α-MEM and 10% FBS that contained 10⁻⁷M dexamethasone, 0.2mM ascorbic acid and 10mM β-gtycero-phosphate (Sigma-Aldrich) for 4 weeks. We used green fluorescent protein (GFP) transgenic rat to isolate mesenchymal stem cells (MSCs) from bone marrow for mesodermic differentiation as a positive control. For neuronal differentiation, cells were plated on fibronectin-coated dishes at a seeding density of 5×10³ cells/cm² in DMEM/F12 supplemented with 5% FBS and 100ng/mL bFGF (PeproTech) for 2 weeks.

In Vivo Differentiation: All procedures involving animals were approved by the Institutional Animal Care and Use Committee of the University of Miami. c-Kit⁺/Lin⁻ cells were labeled with GFP, cultured in EGM-2 for 1 week, and then subcutaneously injected into NOD-SCID mice (2x10⁶ cells) in a MATRIGEL extracellular matrix plug. The MATRIGEL extracellular matrix plugs were removed after 2 weeks for histological analyses.

Regenerative Potential of c-Kit⁺ Cells: Initially, c-Kit⁺/Lin⁻ cells are labeled with green fluorescent protein (GFP) for cell tracking. PCNA (Santa Cruz Biotechnology) detection will be performed to assess tubular proliferation. Ischemia-reperfusion in Sprague-Dawley rats will be performed by applying non-traumatic vascular clamps across both renal pedicles for 30m. Subsequently, c-Kit⁺/Lin⁻ cells will be injected directly into the abdominal aorta, above the renal arteries after application of a vascular clamp to the abdominal aorta below the renal arteries to direct the flow of the inject cells. Saline vehicle will be used as control. Reversal of kidney dysfunction represents the ultimate endpoint to assess functional potency of c-Kit⁺/Lin⁻ cells. Thus, during the period of recovery from surgery, blood creatinine and urea will be monitored at three time-points (2 days, 4 days, and 7 days) after infusion of c-Kit⁺/Lin⁻ cells or saline vehicle. If rats recover kidney function, it will be confirmed that this resulted from the effect of the c-Kit⁺/Lin⁻ cells infused by showing that they were incorporated to the kidney tubules, as well as by the increase of cellular proliferation (PCNA analysis) and decrease of apoptosis (TUNEL assay). After 1 week, kidneys will be harvested for analyses, including RT-PCR, Western blot analysis, and immunofluorescence.

Sphere-Forming Assay: Dissociated single c-Kit⁺ cells obtained from c-Kit sheets and 96-well plates (subclones) were plated on ultra-low attachment 6-well plates (Corning, Costar) at 1×10³ cells/ plate in DMEM-F12 medium containing 20% knockout serum replacer, 10 mM MEM non-essential amino acids, 0.2mM β-mercaptoethanol (GIBCO), L-glutamine (Sigma-Aldrich), and 20ng/ml bFGF. Medium was changed every 3 days. After 12 days, cultures were assessed for nephrosphere number. Nephrospheres were defined as free-floating spheres of > 40µm diameter and results were expressed as a percentage of the plated cells. To assess size and number, spheres were visualized with a Nikon Eclipse TS100 inverted microscopic fitted with a Nikon digital camera image capture system and analyzed with image software.

Acute Kidney Ischemia-Reperfusion Injury: Two-month-old female SD rats weighing 200-250g (Charles River Laboratories) were anesthetized, endotracheally intubated, and placed on mechanical ventilation (2% isoflurane and 100% oxygen). A midline incision was performed and nontraumatic vascular clamps were applied across both renal pedicles for 35m. After removing clamps, reperfusion was visually observed and then 2x10⁶ cells were immediately injected directly into the abdominal aorta above the renal arteries, after application of a vascular clamp to the abdominal aorta below the renal arteries. GFP- labeled c-Kit⁺ cells (2x10⁶ cells) or mesenchymal stem cells (2x10⁶ cells) from GFP-SD rats were also injected in other animal group. Saline was injected in the control group. Blood collection was performed in different time-points: time zero, day 1, day 2, day 4, and day 8 post ischemia-reperfusion injury. Creatinine and blood urea nitrogen (BUN) were measured at each time point (Products Vitros Chemistry). Kidneys were harvested after 8 days for histological analyses.

Morphologic Studies, Immunofluorescence, and Proliferating Cell Nuclear Antigen (PCNA) Index on Kidney Tissue: Acute tubular necrosis (ATN) was assigned by semi-quantitative analysis of each individual variable (i.e., casts, brush border loss, tubular dilation, necrosis, and calcification).

### Example 1

c-Kit⁺ cells were isolated from rat kidney explants by immunopanning and further selected by depleting lineage cells (Lin⁻). 0.9% of positive c-Kit⁺/Lin⁻ cells were detected, which grew in monolayer on plastic and could form aggregates that detached and transitorily grew in suspension. The presence of c-Kit⁺ cells was demonstrated by RT-PCR and indirect immunofluorescence.

RT-PCR detected stem cell markers as well as endothelial, epithelial, mesenchymal, and neuronal markers. In addition, c-Kit⁺/Lin⁻ cells presented clonogenicity as demonstrated by two serial dilutions in 96-well plates. Three clones were cultured and expanded at least 15 passages without evidence of senescence.

Self-renewal of c-Kit⁺/Lin⁻ cells was observed by subculturing the cells until late passages. Telomerase activity was detected during different passages of c-Kit⁺/Lin⁻ cells (P11, P24, P43, P50, P52, P65 and P66), and compared to positive control and neonatal rat kidney.

Regarding the multipotent potential of c-Kit⁺/Lin⁻ cells, the in vitro experiments demonstrated mesenchymal differentiation towards two lineages, adipocyte and osteogenic, as shown by the increase of PPAR-gama and Runx2 by RT-PCR, as well positivity for Oil Red and FABP4 and osteopontin, respectively. Interestingly, late passages of c-Kit⁺/Lin⁻ cells (P72, P74 and P75) also possessed adipogenic and osteogenic potential.

Neuronal differentiation was confirmed by phenotype morphology, increase of neuronal markers (CD56, nestin, β-tubulin III, and neurofilament) by RT-PCR, indirect immunofluorescent detection of β-tubulin III, and colocalization of β-tubulin III and neurofilament.

In vitro endothelial differentiation was demonstrated by culturing c-Kit⁺/Lin⁻ cells for 1 week in endothelial basal medium supplemented with growth factors for 1 week, and by tube formation assay on MATRIGEL extracellular matrix substrate after 24h. Also observed was tube formation with late passages of c-Kit⁺/Lin⁻ cells (P71 and P77). Indirect immunofluorescence showed SMA and vWF expression; colocalization of SMA and vWF was also observed.

For hepatocyte differentiation, c-Kit⁺/Lin⁻ cells were cultured in DMEM with 10% FBS supplemented with FGF-4 and HGF for 2 weeks. They changed morphology towards a cobblestone phenotype. After this time, the cells presented AFP by RT-PCR and albumin expression.

In vivo endothelial differentiation was demonstrated by subcutaneous injection of GFP-labeled c-Kit⁺/Lin⁻ cells into NOD-SCID mice (n=3 mice). Those cells were cultured for one week in endothelial medium and after that embedded in a MATRIGEL extracellular matrix plug (2×10⁶ cells). After two weeks, the plug was removed with the skin and histological analyses were performed. H&E staining showed cells infiltrating the plug. Indirect immunofluorescence showed SMA and vWF expression. The negative control was a plug containing only EGM-2 (n=3 mice). Human umbilical vein endothelial cells (HUVEC) were used as positive control (n=2 mice).

**Table 1. Gene expression of c-Kit⁺/Lin⁻ cells by RT-PCR**

| Stemness markers | Endothelial markers | Epithelial markers | Mesenchymal markers | Neuronal markers |
|---|---|---|---|---|
| Oct4 | CD31 | β-catenin | CD73 | CD56 |
| Sox2 | CD34 | Wnt4 | CD90 | P-3 tubulin |
| c-Myc | vWF | Notch2 | CD105 | Synaptopodin |
| Klf4 | VEGFa | WT-1 | CD146 | Nestin |
| Musashi | SMA | Cytokeratin18 | Vimentin | Neurofilament |
| | | ZO-1 | CD24 | |
| | | Cadherin-6 | CD133 | |
| | | | Six2 | |

### Example 2

### Neonatal rat kidney contain c-Kit⁺ stem cells

Cells expressing the c-Kit epitope on their cell surface were widely distributed in the neonatal kidney. They were located not only in renal papilla, but also in the medulla and the nephrogenic zone. These cells expressed E-cadherin and N-cadherin. c-Kit⁺ cells were located primarily within a laminin-positive membrane, indicating that they are epithelial cells. In contrast, c-Kit did not co-localize with Dolichos biflorus agglutinin (DBA), a marker of ureteric bud and its derivates, or with the Na-Cl co-transporter (NCCT/SLC12A3), a distal tubule marker. But c-Kit co-localized at the apical membrane of epithelial cells of the thick ascending limb (TAL) of Henle's loop with the Na-K-2CI co-transporter (NKCC2/SCL12A1) in both nephrogenic cortex and medulla. Aquaporin-1 (AQP1) did not co-localize with c-Kit. c-Kit⁺ cells were not detected in vessels or glomeruli. Importantly, in the adult rat kidney, c-Kit⁺ cells exhibited identical distribution as found in neonatal rat kidney, e.g., co-localization with NKCC2 in the TAL.

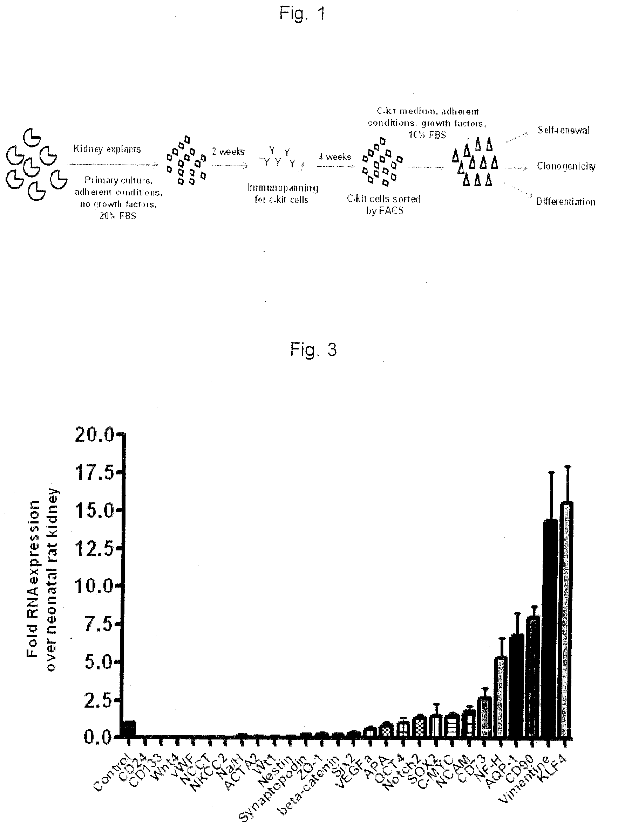

Next, c-Kit⁺ cells were isolated and evaluated for their stemness properties in vitro (see Fig. 1). c-Kit⁺ cells were isolated from cells of kidney explants by immunopanning and fluorescence activated cell sorting. These cells were found to be Lin⁻ (depletion of lineage cells) and represented 1.1% of the cells (∼ 0.15%/kidney). These cells exhibited the ability to self-reflicate and grew in a monolayer on plastic. After sorting, the c-Kit epitope was remained detectable by immunofluorescence microscopy. By FACS, 88.6±5.5% of the cells were positive for c-Kit, and this high level of c-Kit positivity persisted up to 50 passages (76.2± 8.6%).

### Characterization of c-Kit⁺/Lin⁻ cells

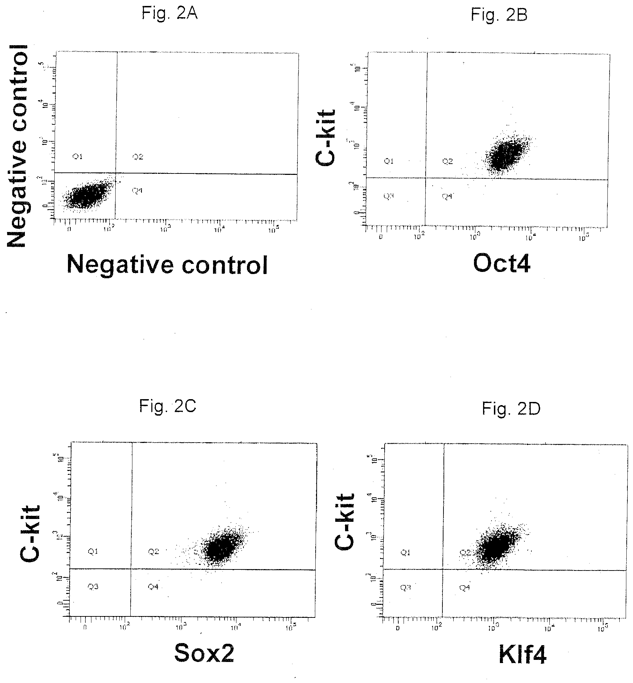

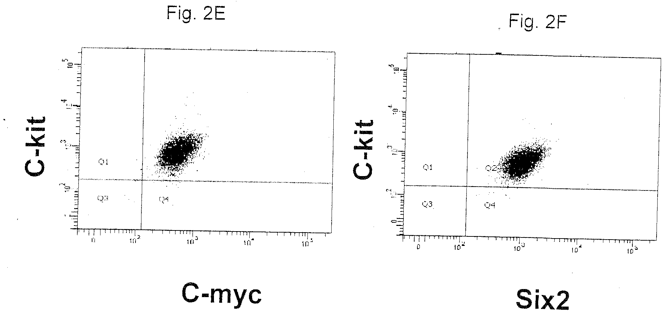

We observed that c-Kit⁺/Lin⁻ cells expressed proteins associated with early stem cells and reprogramming genes (Figs. 2A-2F): octamer-binding POU transcription factor 4 (Oct4), sex-determining-region Y-box 2 (Sox2), basic helix-loop-helix leucine-zipper transcription factor (c-Myc), and Kruppel-like factor 4 (Klf4). A kidney progenitor marker Six2 was also detected in c-Kit⁺ cells. All these markers were confirmed by immunofluorescence staining and qPCR. Vascular (vWF, isolectin, and Acta2), epithelial (ZO-1, NKCC2, NCCT, and AQP1), neuronal (nestin and neurofilament heavy chain), and mesenchymal (CD73, CD90, and vimentin) markers were also detected as RNA (Fig. 3) and protein (Figs. 4A-4J). Although WT-1 was detected by qPCR, no expression was found by immunofluorescence and less than 5% of the cells were stained positive by FACS. Low percentages of kidney-derived c-Kit⁺/Lin⁻ cells expressed CD24 (< 10%), CD133 (∼ 30%), and Pax2 (∼ 30%).

CD73, NF-H, AQP1, CD90, Klf4, and vimentin expression was at least 2.5-fold higher as quantitated by qPCR than neonatal kidney. c-Kit⁺/Lin⁻ cells were negative for CD45, nephrin, poducin, E-cadherin podocin, epithelial cell adhesion molecule (CD326), and mast cell tryptase.

c-Kit⁺/Lin⁻ cells were subcultured for more than a year (> 100 passages) without any evidence of senescence or growth arrest. Cells frozen at different passages and then thawed 6 or 12 months later retained their original characteristics. Similar telomerase activity was detected at different passages of c-Kit⁺/Lin⁻ cells. Moreover, c-Kit⁺/ Lin⁻ cells exhibited a normal karyotype.

### Non-clonal c-Kit⁺-derived cells differentiate into mesoderm and neuroectoderm layers, but not into endoderm

To assess their plasticity, c-Kit⁺ cell monolayers were treated for 1-4 weeks with differentiation media to promote adipogenic, osteogenic, neuronal, epithelial, or endothelial differentiation. The cells successfully differentiated and expressed markers for these cell types, as assessed by histochemical staining, immunostaining, and qPCR.

c-Kit⁺/Lin⁻ cells grown in adipogenic medium for 2 weeks accumulated lipid droplets, that stained positive for Oil-Red O, and up-regulated PPAR-γ and adiponectin. Later passage cells continued to show commitment to adipogenic differentiation, although it was less pronounced. Mesenchymal stem cells (MSCs), the positve control for mesoderm differentiation, significantly exhibited higher lipid accumulation than c-Kit⁺ early and late passage cells. PPAR-γ up-regulation was comparable between c-Kit⁺ cells and MSCs, whereas adiponectin was higher in c-Kit⁺ differentiated cells.

Growing c-Kit⁺/Lin⁻ cells in osteogenic medium for 4 weeks resulted in Alizarin Red S positivity, indicative of mineralization, which correlated with a significant up-regulation of Runx2 and alkaline phosphatase (AP) expression. Osteopontin expression was not significantly up-regulated. Later passages also exhibited Alizarin Red S positivity (data not shown). Similar to adipogenic differentiation, MSCs had more Alizarin Red S staining compared to c-Kit⁺ cells, and a greater up-regulation of Runx2 and osteopontin. At baseline, AP was expressed at low levels in c-Kit⁺ cells, as opposed to MSCs; after differentiation, however, AP up-regulation was more pronounced in c-Kit⁺ cells.

After 2 weeks in the neuronal medium, c-Kit⁺/Lin⁻ cells at low density decreased their proliferation and exhibited prolongations. Cells were positive for β-3 tubulin which co-localized with NF-H. β-3 tubulin was significantly up-regulated.

Epithelial differentiation was induced by growing c-Kit⁺/Lin⁻ cells in medium containing bFGF, TGF-β₃ (Sakurai et al., 1997), and LIF (Barasch et al., 1999) for 3 weeks. After 1 week in epithelial medium, the morphology of c-Kit⁺/Lin⁻ cells changed and they started to form packed clusters. These clusters detached after 3 weeks and acquired an embryoid body-like morphology. Even late passage (P50-P52) cells acquired this morphology. CD24, cytokeratin 18 (KRT18), Wnt4, Notch2, and AQP-1 were all up-regulated, suggesting mesenchymal-epithelial transition (Nishinakamura, 2008; Ivanova et al., 2010). In these epithelial spheres, E-cadherin co-localized with pan-cytokeratin.

### c-Kit vascular differentiation is associated with functional activity in vitro

Based on the presence of vascular markers, we performed in vitro endothelial differentiation by culturing c-Kit⁺/Lin⁻ cells in endothelial basal medium supplemented with growth factors (VEGF, bFGF, IGF-1, and EGF) for 1-4 weeks. Between week 3 and 4, myotube-like structures appeared, which stained for α-actin 2 (Acta2) and co-stained for von Willebrand factor (vWF).

Endothelial tubes were observed at two time-points (6h and 24h) by the in vitro tube formation assay performed on c-Kit⁺/Lin⁻ cells. They were more and longer after 24h compared to 6h. Both early (P15-P20) and late (P50-P71) passage cells formed endothelial tubes on MATRIGEL extracellular matrix subtrate. c-Kit⁺/Lin- cells produced significantly more but shorter tubes compared to MSCs at 24h. In vivo endothelial differentiation demonstrated that GFP-labeled c-Kit⁺/Lin- cells embedded in MATRIGEL extracellular matrix substrate formed network-like connections when injected into NOD-SCID mice. These connections were positive for Acta2 and platelet-endothelial cell adhesion molecule-1 (PECAM-1). HUVEC, a positive control, exhibited pronounced connections in the MATRIGEL extracellular matrix plug, while no connections were seen when a plug containing only EGM-2 was injected.

qPCR data of in vitro endothelial differentiation showed a time-dependent up-regulation of vWF, VEGFa, and desmin (P<0.05), a marginal up-regulation of PECAM-1 (P=0.055), and no significant change in the expression of Acta2. Notch2 and WT-1 genes were also time-dependently regulated. Podocytes markers were not expressed.

After growing c-Kit⁺/Lin⁻ cells in EGM-2 for 1 week, they began to express angiotensin II (Ang II) type 1a (AT1a) receptor and its expression increased significantly with time. In contrast, Ang II type 2 receptor was not detected after differentiation. Calcium (Ca⁺²) gradient analysis demonstrated higher intracellular Ca⁺² concentration in differentiated cells at baseline and their response to extracellular Ca⁺² was more pronounced compared to undifferentiated cells. Therefore, responsiveness to Ang II was assessed. Differentiated cells exhibited higher depolarization following Ang II administration (100nM), a response that was selectively blocked by losartan, but not by PD123319, confirming the involvement of the Ang II type 1a and not Ang II type 2 receptor. Antagonism of inositol-1,4,5-triphosphate (IP3) receptor by 2-aminoethoxydiphenylborane (2-ABP; 60µM) decreased Ca⁺² dependent influx from the sarcoplasmic reticulum after Ang II administration (Stockand & Sansom, 1998). Additionally, differentiated cells failed to respond to isoproterenol or epinephrine, unlike vascular smooth muscle cells (Nobiling & Buhrle, 1987). The dose-response to endothelin via ET_{A} and ET_{B} receptors and to PGF_{2α} was more intense in differentiated compared to undifferentiated cells, a response that was attenuated by 2-ABP, as well as by the specific antagonists BQ-123, BQ-788, and SQ 29,548. The response to bradykinin was more intense in undifferentiated cells and was specifically mediated by B₂ receptor, since HOE 140 decreased the Ca⁺² influx. ET_{A}, PGF_{2α}, B₂ bradykinin receptors were up-regulated with time in endothelial medium. Together these results support the differences in the intracellular Ca⁺² and the responses to extracellular Ca⁺² and to vasoactive agents between undifferentiated and differentiated cells.

### c-Kit⁺/Lin⁻ cells are clonogenic and c-Kit-derived clones exhibit the capacity for multipotent differentiation

To further substantiate the stemness of these cells, clonogenicity was demonstrated. c-Kit⁺/Lin⁻ single cells were obtained by carrying out two serial dilutions in 96-well plates. After obtaining one cell per well, three of the faster proliferating clones were picked and then expanded under non-adherent conditions. All three clones exhibited c-Kit epitope by immunofluorescence.

After growing the c-Kit⁺-derived clones in differentiation media, they all exhibited plasticity. In adipogenic medium, they accumulated lipid droplets that stained for Oil Red O, and exhibited up-regulation of PPAR-γ and adiponectin. In osteogenic medium, Runx2, osteopontin, and AP were significantly up-regulated, and cultures stained for Alizarin Red S. In epithelial medium, embryoid body-like morphology was observed, and the genes involved in epithelial commitment were up-regulated. Co-localization of pan-cytokeratin and E-cadherin was also observed in the epithelial spheres.

### c-Kit⁺ cells form neprospheres when grown in non-adherent conditions

Sphere-forming assays have been used, both retrospectively and prospectively, to investigate stem cells and precursors in many tissues during development and in the adult (Pastrana et al., 2011). A central tenet of sphere-forming assays is that each sphere is derived from a single cell and is therefore clonal.

For that purpose, non-clonal (non-single cell derived) and clonal (single cell-derived) c-Kit⁺ cells, previously grown in adherent conditions, were dissociated into single cells and grown at clonal density (1x10³ cells/well), in 6-well plates, in non-adherent conditions. Primary spheres were formed by proliferation instead of aggregation and were visible after 4 days at a frequency of ∼ 2.5% of the initial plated cells. Accordingly, these cells clearly are clonogenic supporting their identity as a true stem cell. The majority of spheres were small, measuring 40µm-100µm. The spheres were passaged a minimum of three times, demonstrating self-renewal capacity. But the higher proliferation rate was observed when plating cells in adherent conditions, likely reflecting the importance of cell-cell interaction and cell adhesion for c-Kit⁺/Lin⁻ cell growth.

c-Kit-derived spheres exhibited markers from both neuroectoderm and mesoderm lineages, including nestin, β-3 tubulin, Acta2, isolectin, pan-cytokeratin, E-cadherin, and other markers found in the kidney (NKCC2, NCCT, and AQP1). Co-localization of c-Kit receptor and NKCC2 was observed in the spheres. Up-regulation of those genes was observed in primary and secondary spheres.

### Regenerative capacity of c-Kit⁺/Lin⁻ cells after acute ischemia-reperfusion injury

As a final test of the regeneration capacity of kidney neonatal c-Kit⁺ stem cells, we assessed their ability for in vivo tissue repair. To evaluate the potential of c-Kit⁺/Lin⁻ cells to improve renal function in vivo, we utilized the model of acute ischemia-reperfusion injury (IRI) (Togel et al., 2005). This model which mainly affects proximal tubular function, also affects the glomeruli involving podocyte foot process effacement (Wagner et al., 2008).

c-Kit⁺/Lin⁻ cells (n=8), MSCs (n=6), or saline (n=12) was injected following IRI into the aorta immediately upstream of the renal arteries, while gently clamping the aorta below the kidneys. Animals were followed for 8 days. c-Kit⁺/Lin⁻ cells promoted renal function recovery as demonstrated by improvement of creatinine and BUN at day 4. MSCs improved renal function at day 2. c-Kit⁺/Lin⁻ cells and MSCs-treated animals exhibited not only a less severe kidney injury score, but also a higher proliferation of surviving epithelial tubular cells in comparison to the control, as indicated by less tubular damage compared to the control group.

Immunofluorescence staining with an anti-GFP antibody and staining for E-cadherin indicated that c-Kit⁺/Lin⁻ cells were integrated into tubules in all 8 animals studied. c-Kit⁺/Lin⁻ cells also engrafted into glomeruli and vessels in 3 of 8 animals. Most of GFP-labeled c-Kit⁺ cells engrafted into glomeruli were found in Bowman's capsule, while a few of them were also seen in podocytes, as demonstrated by the co-localization with WT-1. In the MSC group, tubular engraftment was observed in all six cases, but only one case exhibited glomerular engraftment. There were also GFP⁺-cells observed within the lumen of the tubules, indicating that some cells may have been eliminated in the urine.

**Table 2. Gene expression of c-Kit⁺/Lin⁻ cells by qPCR, quantitated by the mean Cₜ (threshold cycle)**

| Cₜ < 20 | 20 < Cₜ ≤ 25 | 25 < Cₜ ≤ 30 | 30 < Cₜ ≤ 35 | 35 < Cₜ ≤ 39 |
|---|---|---|---|---|
| Vimentin | VEGFa | vWF | c-Kit (CD117) | Wnt4 |
| | β-catenin | Acta2 | PECAM-1 | KRT18 |
| | Notch2 | WT-1 | CD34 | β-3 tubulin |
| | Klf4 | Sox2 | ZO-1 | CD24 |
| | CD90 | c-Myc | Oct4 | |
| | Synaptopodin | CD56 | CD133 | |
| | ET type Ia receptor | CD73 | NF-H | |
| | Aquaporin-1 | CD105 | Desmin | |
| | | CD146 | Bradykinin B₁ receptor | |
| | | Six2 | Bradykinin B₂ receptor | |
| | | Nestin | | |
| | | PGF_{2α} | | |
| | | receptor | | |

| | | | | |
|---|---|---|---|---|
| ET is endothelin, PG is prostaglandin, NF-H is neurofilament heavy chain, and KRT18 is keratin 18. | | | | |

Here, we demonstrate that c-Kit⁺ cells originating from neonatal kidneys are a novel population of stem cells. They exhibit the fundamental properties of stem cells, including clonogenicity, self-renewal, multipotent capacity for commitment to mesoderm and neuroectoderm lineages, and contribute to kidney repair through multi-compartment engraftment.

During neonatal rat kidney development, intense proliferation is seen in s-shaped bodies, immature tubules, and undifferentiated cells (Marquez et al., 2002). Several genes are up-regulated during that period, including the c-Kit receptor in both MM and ureteric bud (Schmidt-Ott et al., 2005). Additionally, exogenous SCF expands c-Kit⁺ population from both renal interstitium and hemangioblasts, accelerating kidney development (Schmidt-Ott et al., 2006). Studies on transgenic mice confirmed c-Kit expression in hemangioblasts, MM, and additionally in the epithelial cells of distal tubules, collecting ducts, ureter and bladder (Bernex et al., 1996).

We detected c-Kit⁺ cells in the TAL, a MM-derived structure, where these cells exhibited characteristics of MM, including NCAM (CD56), Six2, and WT-1 (Oliver et al., 2002; Markovic-Lipkovski et al., 2007; Metsuyanim et al., 2009). MM-derived cells also express epithelial, mesenchymal, endothelial, neuronal, and renal differentiated cell markers (Oliver et al., 2002; Nishinakamura, 2008; Metsuyanim et al., 2009; Batchelder et al., 2010), as did our neonatal c-Kit⁺ cells. Notably, immature tubules can also express vimentin and epithelial markers, such as ZO-1. However, Six2/WT-1 expression in the c-Kit⁺ cells is intriguing, because we did not find co-localization in paraffin-embedded sections. One explanation could be that c-Kit⁺ cells may represent a cellular kidney subfraction that can be induced to return to cap mesenchyma-like structures upon isolation. Early stem cells and reprogramming genes Oct4, Sox2, Klf4, and c-Myc are detected in developing kidneys according to the GUDMAP database (Harding et al., 2011). This is interesting in light of studies showing that inducible pluripotent stem cells were obtained from proximal tubular cells having only two transcription factors (Oct4 and Sox2) (Montserrat et al., 2012) or four transcription factors (Oct4, Sox2, Klf4, and c-Myc) (Song et al., 2011a) from mesangial cells, suggesting that epigenetic memory might also exist in the kidney. Oct4 is dispensable, however, for both maintenance and self-renewal of somatic stem cells in the adult mammal (Lengner et al., 2007). Furthermore, Klf4 regulates kidney epithelial tubular differentiation (Saifudeen et al., 2005), while c-Myc promotes proliferation of renal progenitors (Couillard and Trudel, 2009).

Sphere-forming assays were tested in different adult murine and human tissues, including anterior pituitary, prostate, dermis, pancreas, cornea, retina, breast, and heart (Pastrana et al., 2011), but not in kidney. They became a useful tool to test the potential of cells to exhibit stem cells traits, although not considered a read-out of in vivo stem cell activity. Here, renal stem cell-derived nephrospheres exhibited markers of neuroectoderm and mesoderm progeny. It is noteworthy that common sphere features include the presence of stem, precursor, and differentiated cells, the expression of nestin, routinely used for detection of neural stem cells but also characteristic for progenitor epithelial cells, and the ability of the sphere-derived cells to differentiate into other cell types in addition to their own tissue-specific cell type. More recently, E-cadherin and keratin 18 were described as early differentiation markers in embryonic stem cells (Galat et al., 2012), although contrasting data showed E-cadherin involvement in somatic cell reprogramming (Redmer et al., 2011).

In the present study, renal stem cell-mediated kidney regeneration involved multi-compartment engraftment. Importantly, our study does not rule out a paracrine effect (Perin et al., 2010) or the intrinsic mechanism of repair due to the proliferating capacity of surviving tubular epithelial cells (Vogetseder et al., 2008; Humphreys et al., 2008). Further lineage tracing studies could evaluate the involvement c-Kit⁺ cells in that mechanism. Engraftment of c-Kit⁺ cells into Bowman's capsule and podocytes suggests that these cells may also play a role in repopulating kidney stem cell niches, as the one described in Bowman's capsule (Lazzeri et al., 2007; Ronconi et al., 2009). Furthermore, c-Kit⁺ cells from different organs, including biliary (Crosby et al., 2001), bronchiolar (Kajstura et al., 2011), and renal epithelia (Perin et al., 2007) exhibit stem cell characteristics and epithelial differentiation. Moreover, c-Kit⁺ cells can promote kidney regeneration by an autocrine mechanism, as demonstrated by the shift of these cells from the papilla and medullary rays to the corticomedullary area following acute ischemia-reperfusion injury (Stokman et al., 2010). c-Kit⁺ cells described here, however, are distinct from the kidney c-Kit⁺ side population, because the latter exhibited variable differentiation potential and failed to integrate into tubules (Iwatani et al., 2004; Challen et al., 2006).

A claim using the transitional "compri-sing" allows the inclusion of other elements to be within the scope of the claim; the invention is also described by such claims using the transitional phrase "consisting essentially of" (i.e., allowing the inclu-sion of other elements to be within the scope of the claim if they do not materially affect operation of the invention) and the transition "consisting" (i.e., allowing only the elements listed in the claim other than impurities or inconsequential activities which are ordinarily associated with the invention) instead of the "comprising" term. Any of these three transitions can be used to claim the invention.

It should be understood that an element described in this specification should not be construed as a limitation of the claimed invention unless it is explicitly recited in the claims. Thus, the granted claims are the basis for determining the scope of legal protection instead of a limitation from the specification which is read into the claims.

No particular relationship between or among limitations of a claim is intended unless such relationship is explicitly recited in the claim (e.g., the arrangement of components in a product claim or order of steps in a method claim is not a limitation of the claim unless explicitly stated to be so).

From the foregoing, it would be apparent to a person of skill in this art that the invention can be embodied in other specific forms without departing from its spirit or essential characteristics. The described embodiments should be considered only as illustrative, not restrictive, because the scope of the legal protection provided for the invention will be indicated by the appended claims rather than by this specification.

### References

Alvarez-Buylla et al. (2002) Identification of neural stem cells in the adult vertebrate brain. Brain Res. Bull. 57, 751-758.
Appel et al. (2009) Recruitment of podocytes from glomerular parietal epithelial cells. J. Am. Soc. Nephrol. 20, 333-343.
Barasch et al. (1999) Mesenchymal to epithelial conversion in rat metanephros is induced by LIF. Cell 99, 377-386.
Batchelder et al. (2010) Ontogeny of the kidney and renal developmental markers in the rhesus monkey (Macaca mulatta) Anat. Rec. (Hoboken) 293, 1971-1983.
Beltrami et al. (2003) Adult cardiac stem cells are multipotent and support myocardial regeneration. Cell 114, 763-776.
Bernex et al. (1996) Spatial and temporal patterns of c-Kit-expressing cells in WlacZ/+ and WlacZ/WlacZ mouse embryos. Development 122, 3023-3033.
Bussolati et al. (2005) Isolation of renal progenitor cells from adult human kidney. Am. J. Pathol. 166, 545-555.
Challen et al. (2006) Kidney side population reveals multilineage potential and renal functional capacity but also cellular heterogeneity. J. Am. Soc. Nephrol. 17, 1896-1912.
Couillard & Trudel (2009) c-Myc as a modulator of renal stem/progenitor cell population. Dev. Dyn. 238, 405-414.
Crosby et al. (2001) Human hepatic stem-like cells isolated using c-Kit or CD34 can differentiate into biliary epithelium. Gastroenterology 120, 534-544.
De et al. (2007) Isolation of amniotic stem cell lines with potential for therapy. Nat. Biotechnol. 25, 100-106.
Dekel et al. (2006) Isolation and characterization of nontubular sca-1+lin- multipotent stem/progenitor cells from adult mouse kidney. J. Am. Soc. Nephrol. 17, 3300-3314.
Dor et al. (2004) Adult pancreatic beta-cells are formed by self-duplication rather than stem-cell differentiation. Nature 429, 41-46.
Galat et al. (2012) A model of early human embryonic stem cell differentiation reveals inter- and intracellular changes on transition to squamous epithelium. Stem Cells Dev. 21, 1250-1263.
Harding et al. (2011) The GUDMAP database - An online resource for genitourinary research. Development 138, 2845-2853.
Hishikawa et al. (2005) Musculin/MyoR is expressed in kidney side population cells and can regulate their function. J. Cell Biol. 169, 921-928.
Humphreys et al. (2011) Repair of injured proximal tubule does not involve specialized progenitors. Proc. Natl. Acad. Sci. USA 108, 9226-9231.
Humphreys et al. (2008) Intrinsic epithelial cells repair the kidney after injury. Cell Stem Cell 2, 284-291.
Ivanova et al. (2010) Ontogeny of CD24 in the human kidney. Kidney Int. 77, 1123-1131.
Iwatani et al. (2004) Hematopoietic and nonhematopoietic potentials of Hoechst(low)/side population cells isolated from adult rat kidney. Kidney Int. 65, 1604-1614.
Kajstura et al. (2011) Evidence for human lung stem cells. N. Engl. J. Med. 364, 1795-1806.
Lazzeri et al. (2007) Regenerative potential of embryonic renal multipotent progenitors in acute renal failure. J. Am. Soc. Nephrol. 18, 3128-3138.
Lengner et al. (2007) Oct4 expression is not required for mouse somatic stem cell self-renewal. Cell Stem Cell 1, 403-415.
Lin et al. (2005) Intrarenal cells, not bone marrow-derived cells, are the major source for regeneration in postischemic kidney. J. Clin. Invest 115, 1756-1764.
Lindgren et al. (2011) Isolation and characterization of progenitor-like cells from human renal proximal tubules. Am. J. Pathol. 178, 828-837.
Maeshima et al. (2006) Adult kidney tubular cell population showing phenotypic plasticity, tubulogenic capacity, and integration capability into developing kidney. J. Am. Soc. Nephrol. 17, 188-198.
Maeshima et al. (2003) Identification of renal progenitor-like tubular cells that participate in the regeneration processes of the kidney. J. Am. Soc. Nephrol. 14, 3138-3146.
Markovic-Lipkovski et al. (2007) Neural cell adhesion molecule expression on renal interstitial cells. Nephrol. Dial. Transplant. 22, 1558-1566.
Marquez et al. (2002) Cell proliferation and morphometric changes in the rat kidney during postnatal development. Anat. Embryol. (Berl) 205, 431-440.
Metsuyanim et al. (2009) Expression of stem cell markers in the human fetal kidney. PLoS One 4, e6709.
Miettinen & Lasota (2005) KIT (CD117): A review on expression in normal and neoplastic tissues, and mutations and their clinicopathologic correlation. Appl. Immunohistochem. Mol. Morphol. 13, 205-220.
Montserrat et al. (2012) Generation of induced pluripotent stem cells from human renal proximal tubular cells with only two transcription factors, Oct4 and Sox2. J. Biol. Chem. 287, 24131-24138.
Nishinakamura (2008) Stem cells in the embryonic kidney. Kidney Int. 73, 913-917.
Nobiling & Buhrle (1987) The mesangial cell culture: A tool for the study of the electrophysiological and pharmacological properties of the glomerular mesangial cell. Differentiation 36, 47-56.
Ogawa et al. (1993) Expression and function of c-Kit in fetal hemopoietic progenitor cells: Transition from the early c-Kit-independent to the late c-Kit-dependent wave of hemopoiesis in the murine embryo. Development 117, 1089-1098.
Oliver et al. (2002) Metanephric mesenchyme contains embryonic renal stem cells. Am. J. Physiol. Renal Physiol. 283, F799-F809.
Oliver et al. (2009) Proliferation and migration of label-retaining cells of the kidney papilla. J. Am. Soc. Nephrol. 20, 2315-2327.
Oliver et al. (2012) SDF-1 activates papillary label-retaining cells during kidney repair from injury. Am. J. Physiol. Renal Physiol. 302, F1362-F1373.
Oliver et al. (2004) The renal papilla is a niche for adult kidney stem cells. J. Clin. Invest. 114, 795-804.
Pastrana et al. (2011) Eyes wide open: A critical review of sphereformation as an assay for stem cells. Cell Stem Cell 8, 486-498.
Perin et al. (2007) Renal differentiation of amniotic fluid stem cells. Cell Prolif. 40, 936-948.
Perin et al. (2010) Protective effect of human amniotic fluid stem cells in an immunodeficient mouse model of acute tubular necrosis. PLoS One 5, e9357.
Redmer et al. (2011) E-cadherin is crucial for embryonic stem cell pluripotency and can replace Oct4 during somatic cell reprogramming. EMBO Rep. 12, 720-726.
Ronconi et al. (2009) Regeneration of glomerular podocytes by human renal progenitors. J. Am. Soc. Nephrol. 20, 322-332.
Sagrinati et al. (2006) Isolation and characterization of multipotent progenitor cells from the Bowman's capsule of adult human kidneys. J. Am. Soc. Nephrol. 17, 2443-2456.
Saifudeen et al. (2005) Combinatorial control of the bradykinin B2 receptor promoter by p53, CREB, KLF-4, and CBP: Implications for terminal nephron differentiation. Am. J. Physiol. Renal Physiol. 288, F899-F909.
Sakurai et al. (1997) An in vitro tubulogenesis system using cell lines derived from the embryonic kidney shows dependence on multiple soluble growth factors. Proc. Natl. Acad. Sci. USA 94, 6279-6284.
Sallustio et al. (2010) TLR2 plays a role in the activation of human resident renal stem/progenitor cells. FASEB J. 24, 514-525.
Schmidt-Ott et al. (2005) Novel regulators of kidney development from the tips of the ureteric bud. J. Am. Soc. Nephrol. 16, 1993-2002.
Schmidt-Ott et al. (2006) c-Kit delineates a distinct domain of progenitors in the developing kidney. Dev. Biol. 299, 238-249.
Smukler et al. (2011) The adult mouse and human pancreas contain rare multipotent stem cells that express insulin. Cell Stem Cell 8, 281-293.
Song et al. (2011a) Generation of induced pluripotent stem cells from human kidney mesangial cells. J. Am. Soc. Nephrol. 22, 1213-1220.
Song et al. (2011b) Characterization and fate of telomerase-expressing epithelia during kidney repair. J. Am. Soc. Nephrol. 22, 2256-2265.
Stockand & Sansom (1998) Glomerular mesangial cells: Electrophysiology and regulation of contraction. Physiol. Rev. 78, 723-744.
Stokman et al. (2010) Stem cell factor expression after renal ischemia promotes tubular epithelial survival. PLoS One 5, e14386.
Togel et al. (2005) Administered mesenchymal stem cells protect against ischemic acute renal failure through differentiation-independent mechanisms. Am. J. Physiol. Renal Physiol. 289, F31-F42.
Vogetseder et al. (2008) Proliferation capacity of the renal proximal tubule involves the bulk of differentiated epithelial cells. Am. J. Physiol. Cell Physiol. 294, C22-C28.
Wagner et al. (2008) Ischemic injury to kidney induces glomerular podocyte effacement and dissociation of slit diaphragm proteins Neph1 and ZO-1. J. Biol. Chem. 283, 35579-35589.
Ward et al. (2011) Adult human CD133/1(+) kidney cells isolated from papilla integrate into developing kidney tubules. Biochim. Biophys. Acta 1812, 1344-1357.

## Claims

1. An in vitro process of producing c-Kit⁺/Lin⁻ renal stem cells that express Oct4, which are multipotent precursors of endothelial, epithelial, mesenchymal, and neuronal cells the process comprising:
(a) isolating c-Kit⁺/Lin⁻ cells derived from kidney tissue wherein Lin⁻ cells are negative for hematopoietic lineage markers; and
(b) expanding the c-Kit⁺/Lin⁻ cells by in vitro cell culture, wherein the renal stem cells express Oct4.

2. The process of claim 1, further comprising removing cells identified by the markers selected from the group consisting of CD3e, CD11b, CD45R/B220, Ly-76, Ly-6G, and Ly-6C from cells isolated or derived from kidney tissue.

3. The process according to claim 1 or 2, wherein the renal stem cell further expresses at least one endothelial cell lineage marker CD31, CD34, Acta2, isolectin, vWF, VEGFa, SMA, or a combination thereof.

4. The process according to claim 1 or 2, wherein the renal stem cell further expresses at least one epithelial cell lineage marker Wnt4, NKCC2, NCCT, WT-1, cytokeratin 18, ZO-1, β-catenin, Notch2, AQP1, cadherin 6, or a combination thereof.

5. The process according to claim 1 or 2, wherein the renal stem cell further expresses at least one mesenchymal cell lineage marker CD73, CD90, CD105, CD146, vimentin, CD24, CD133, Six2, or a combination thereof.

6. The process according to claim 1 or 2, wherein the renal stem cell further expresses at least one neuronal cell lineage marker CD56, β-3 tubulin, synaptopodin, nestin, neurofilament, or a combination thereof.

7. The process according to claim 1 or 2, wherein the renal stem cell does not detectably express at least one marker CD45, nephrin, poducin, E-cadherin podocin, CD326, angiotensin receptor type Ia, angiotensin receptor type II, mast cell tryptase, or a combination thereof.

8. The process according to any one of claims 1-7, comprising isolating c-Kit⁺/Lin⁻ cells derived from neonatal kidney tissue.

9. c-Kit⁺/Lin⁻ renal stem cells that express Oct4, which are multipotent precursors of endothelial, epithelial, mesenchymal, and neuronal cells.

10. The stem cells of claim 9, wherein the c-Kit⁺/Lin⁻ cells:
(A) further express at least one endothelial cell lineage marker, optionally, CD31, CD34, Acta2, isolectin, vWF, VEGFa, SMA, or a combination thereof;
(B) further expresses at least one epithelial cell lineage marker, optionally, Wnt4, NKCC2, NCCT, WT-1, cytokeratin 18, ZO-1, β-catenin, Notch2, AQP1, cadherin 6, or a combination thereof;
(C) further expresses at least one mesenchymal cell lineage marker, optionally, CD73, CD90, CD105, CD146, vimentin, CD24, CD133, Six2, or a combination thereof;
(D) further expresses at least one neuronal cell lineage marker, optionally, CD56, β-3 tubulin, synaptopodin, nestin, neurofilament, or a combination thereof;
(E) do not detectably express at least one or more of the following markers: CD45, nephrin, poducin, E-cadherin podocin, CD326, angiotensin receptor type Ia, angiotensin receptor type II, mast cell tryptase;
(F) do not detectably express at least one or more of the following markers: CD3e, CD11b, CD45R/B220, Ly-76, Ly-6G, and Ly-6C; or
(G) or a combination thereof.

11. The stem cells of claim 9 or 10, wherein the c-Kit⁺/Lin⁻ stem cells exhibit self-renewal capacity, clonogenicity, capability of engrafting in multiple compartments of the kidney to promote tissue repair.

12. An in vitro purified cell composition comprising at least 50% renal stem cells according to claims 9-11.

13. The composition of claim 12, which is comprised of at least 10⁷ renal stem cells per milliliter or at least 10⁶ renal stem cells.

14. The composition of claim 12 or 13 for use in treating a kidney disease or disorder, or repairing or regenerating kidney tissue in vivo.

15. The composition of any one of claims 10-14, wherein the c-Kit⁺/Lin⁻ cells are derived from neonatal kidney tissue.

## Patentansprüche

1. *In-vitro-*Verfahren zum Herstellen von C-Kit⁺/Lin⁻-Nierenstammzellen, die Oct4 exprimieren, die multipotente Vorläufer von endothelialen, epithelialen, mesenchymalen und neuronalen Zellen sind, wobei das Verfahren Folgendes umfasst:
(a) Isolieren von aus Nierengewebe gewonnenen c-Kit⁺/Lin⁻-Zellen, wobei Lin⁻-Zellen für hämatopoetische Zelltypmarker negativ sind; und
(b) Vergrößern der c-Kit⁺/Lin⁻-Zellen durch *in*-*vitro*-Zellkultur, wobei die Nierenstammzellen Oct4 exprimieren.

2. Verfahren nach Anspruch 1, ferner umfassend das Entfernen der Zellen, die durch die Marker, ausgewählt aus der Gruppe, bestehend aus CD3e, CD11b, CD45R/B220, Ly-76, Ly-6G und Ly-6C, identifiziert wurden, aus Zellen, die aus Nierengewebe isoliert oder gewonnen wurden.

3. Verfahren nach Anspruch 1 oder 2, wobei die Nierenstammzelle ferner mindestens einen Zelltypmarker für endotheliale Zellen CD31, CD34, Acta2, Isolectin, vWF, VEGFa, SMA oder eine Kombination davon exprimiert.

4. Verfahren nach Anspruch 1 oder 2, wobei die Nierenstammzelle ferner mindestens einen Zelltypmarker für epitheliale Zellen Wnt4, NKCC2, NCCT, WT-1, Cytokeratin 18, ZO-1, β-Catenin, Notch2, AQP1, Cadherin 6 oder eine Kombination davon exprimiert.

5. Verfahren nach Anspruch 1 oder 2, wobei die Nierenstammzelle ferner mindestens einen Zelltypmarker für mesenchymale Zellen CD73, CD90, CD105, CD146, Vimentin, CD24, CD133, Six2 oder eine Kombination davon exprimiert.

6. Verfahren nach Anspruch 1 oder 2, wobei die Nierenstammzelle ferner mindestens einen Zelltypmarker für neuronale Zellen CD56, β-3-Tubulin, Synaptopodin, Nestin, Neurofilament oder eine Kombination davon exprimiert.

7. Verfahren nach Anspruch 1 oder 2, wobei die Nierenstammzelle mindestens einen Marker CD45, Nephrin, Poducin, E-Cadherin Podocin, CD326, Angiotensin-Rezeptor Typ la, Angiotensin-Rezeptor Typ II, Mastzellen-Tryptase oder eine Kombination davon nicht nachweisbar exprimiert.

8. Verfahren nach einem der Ansprüche 1 bis 7, umfassend das Isolieren von aus neonatalem Nierengewebe gewonnenen c-Kit⁺/Lin⁻-Zellen.

9. c-Kit⁺/Lin⁻-Nierenstammzellen, die Oct4 exprimieren, die multipotente Vorläufer von endothelialen, epithelialen, mesenchymalen und neuronalen Zellen sind.

10. Isolierte Stammzellen nach Anspruch 9, wobei die c-Kit⁺/Lin⁻-Zellen:
(A) ferner mindestens einen Zelltypmarker für endotheliale Zellen exprimieren, optional CD31, CD34, Acta2, Isolectin, vWF, VEGFa, SMA oder eine Kombination davon;
(B) ferner mindestens einen Zelltypmarker für epitheliale Zellen exprimieren, optional Wnt4, NKCC2, NCCT, WT-1, Cytokeratin 18, ZO-1, β-Catenin, Notch2, AQP1, Cadherin 6 oder eine Kombination davon;
(C) ferner mindestens einen Zelltypmarker für mesenchymale Zellen exprimieren, optional CD73, CD90, CD105, CD146, Vimentin, CD24, CD133, Six2 oder eine Kombination davon;
(D) ferner mindestens einen Zelltypmarker für neuronale Zellen exprimieren, optional CD56, β-3-Tubulin, Synaptopodin, Nestin, Neurofilament oder eine Kombination davon;
(E) mindestens einen oder mehrere der folgenden Marker nicht nachweisbar exprimieren: CD45, Nephrin, Poducin, E-Cadherin Podocin, CD326, Angiotensin-Rezeptor Typ la, Angiotensin-Rezeptor Typ II, Mastzell-Tryptase;
(F) mindestens einen oder mehrere der folgenden Marker nicht nachweisbar exprimieren: CD3e, CD11b, CD45R/B220, Ly-76, Ly-6G und Ly-6C;
(G) oder eine Kombination davon.

11. Stammzellen nach Anspruch 9 oder 10, wobei die c-Kit⁺/Lin⁻-Stammzellen Selbsterneuerungskapazität, Klonogenizität, Transplantationsfähigkeit in mehreren Kompartimenten der Niere, um Gewebereparatur zu fördern, zeigen.

12. *In*-*vitro*-gereinigte Zellzusammensetzung, umfassend mindestens 50 % Nierenstammzellen nach den Ansprüchen 9 bis 11.

13. Zusammensetzung nach Anspruch 12, die aus mindestens 10⁷ Nierenstammzellen pro Milliliter oder mindestens 10⁶ Nierenstammzellen besteht.

14. Zusammensetzung nach Anspruch 12 oder 13 zur Verwendung bei der Behandlung einer Nierenerkrankung oder -störung oder zum Reparieren oder Regenerieren von Nierengewebe *in vivo.*

15. Zusammensetzung nach einem der Ansprüche 10 bis 14, wobei die c-Kit⁺/Lin⁻-Zellen aus neonatalem Nierengewebe gewonnen werden.

## Revendications

1. Procédé in vitro de production de cellules souches rénales c-Kit⁺/Lin⁻ exprimant Oct4, qui sont des précurseurs multipotents de cellules endothéliales, épithéliales, mésenchymateuses et neuronales, le procédé comprenant :
(a) l'isolation de cellules c-Kit⁺/Lin⁻ dérivées de tissu rénal dans lesquelles les cellules Lin⁻ sont négatives pour les marqueurs de lignées hématopoïétiques ; et
(b) l'expansion des cellules c-Kit⁺/Lin⁻ par culture cellulaire in vitro, dans laquelle les cellules souches rénales expriment Oct4.

2. Procédé selon la revendication 1, comprenant en outre le prélèvement de cellules identifiées par les marqueurs choisis dans le groupe constitué de CD3e, CD11b, CD45R/B220, Ly-76, Ly-6G et Ly-6C à partir de cellules isolées ou dérivées de tissu rénal.

3. Procédé selon la revendication 1 ou 2, dans lequel la cellule souche rénale exprime en outre au moins un marqueur de lignée de cellules endothéliales parmi CD31, CD34, ACTA2, l'isolectine, vWF, VEGFA, SMA ou une combinaison de ceux-ci.

4. Procédé selon la revendication 1 ou 2, dans lequel la cellule souche rénale exprime en outre au moins un marqueur de lignée de cellules épithéliales parmi Wnt4, NKCC2, NCCT, WT-1, la cytokératine 18, ZO-1, la β-caténine, Notch2, AQP1, la cadhérine 6 ou une combinaison de ceux-ci.

5. Procédé selon la revendication 1 ou 2, dans lequel la cellule souche rénale exprime en outre au moins un marqueur de lignée de cellules mésenchymateuses parmi CD73, CD90, CD105, CD146, la vimentine, CD24, CD133, Six2 ou une combinaison de ceux-ci.

6. Procédé selon la revendication 1 ou 2, dans lequel la cellule souche rénale exprime en outre au moins un marqueur de lignée de cellules neuronales parmi CD56, la tubuline β-3, la synaptopodine, la nestine, le neurofilament ou une combinaison de ceux-ci.

7. Procédé selon la revendication 1 ou 2, dans lequel la cellule souche rénale n'exprime pas de manière détectable au moins un marqueur parmi CD45, la néphrine, la poducine, la podocine cadhérine E, CD326, le récepteur de l'angiotensine de type la, le récepteur de l'angiotensine de type II, la tryptase des mastocytes ou une combinaison de ceux-ci.

8. Procédé selon l'une quelconque des revendications 1 à 7, comprenant l'isolation de cellules c-Kit⁺/Lin⁻ dérivées de tissu rénal néonatal.

9. Cellules souches rénales c-Kit⁺/Lin⁻ exprimant Oct4, qui sont des précurseurs multipotents de
cellules endothéliales, épithéliales, mésenchymateuses et neuronales.

10. Cellules souches isolées selon la revendication 9, dans lesquelles les cellules c-Kit⁺/Lin⁻:
(A) expriment en outre au moins un marqueur de lignée de cellules endothéliales parmi, facultativement, CD31, CD34, Acta2, l'isolectine, vWF, VEGFa, SMA ou une combinaison de ceux-ci ;
(B) expriment en outre au moins un marqueur de lignée de cellules épithéliales parmi, facultativement, Wnt4, NKCC2, NCCT, WT-1, la cytokératine 18, ZO-1, la β-caténine, Notch2, AQP1, la cadhérine 6 ou une combinaison de ceux-ci ;
(C) expriment en outre au moins un marqueur de lignée de cellules mésenchymateuses parmi, facultativement, CD73, CD90, CD105, CD146, la vimentine, CD24, CD133, Six2 ou une combinaison de ceux-ci ;
(D) expriment en outre au moins un marqueur de lignée de cellules neuronales parmi, facultativement, CD56, la tubuline β-3, la synaptopodine, la nestine, le neurofilament ou une combinaison de ceux-ci ;
(E) n'expriment pas de manière détectable au moins un ou plusieurs des marqueurs suivants : CD45, néphrine, poducine, podocine cadhérine E, CD326, récepteur de l'angiotensine de type la, récepteur de l'angiotensine de type II, tryptase des mastocytes ;
(F) n'expriment pas de manière détectable au moins un ou plusieurs des marqueurs suivants : CD3e, CD11b, CD45R/B220, Ly-76, Ly-6G et Ly-6C ;
(G) ou une combinaison de ceux-ci.

11. Cellules souches selon la revendication 9 ou 10, dans lesquelles les cellules souches c-Kit⁺/Lin⁻ présentent une capacité d'auto-renouvellement, une clonogénicité, une capacité de prise de greffe dans des compartiments multiples du rein pour promouvoir la réparation tissulaire.

12. Composition cellulaire purifiée in vitro comprenant au moins 50 % de cellules souches rénales selon les revendications 9 à 11.

13. Composition selon la revendication 12, qui est constituée d'au moins 10⁷ cellules souches rénales par millilitre ou d'au moins 10⁶ cellules souches rénales.

14. Composition selon la revendication 12 ou 13 pour une utilisation dans le traitement d'une maladie rénale ou d'un trouble rénal, ou dans la réparation ou la régénération de tissu rénal in vivo.

15. Composition selon l'une quelconque des revendications 10 à 14, dans laquelle les cellules c-Kit⁺/Lin⁻ sont dérivées de tissu rénal néonatal.
